# EUROPEAN PATENT SPECIFICATION

(11) **EP 1 833 988 B1**
(45) Date of publication and mention of the grant of the patent: **14.08.2013**
(21) Application number: 05820627.7
(22) Date of filing: 23.12.2005
(51) Int. Cl.: C12Q 1/68

(54) **NOVEL GENES AND MARKERS ASSOCIATED TO TYPE 2 DIABETES MELLITUS**
NEUE, MIT DIABETES MELLITUS TYP 2 ASSOZIIERTE GENE UND MARKER
NOUVEAUX GENES ET MARQUEURS ASSOCIES AU DIABETE SUCRE DE TYPE 2

(30) Priority: 05.01.2005 FI 20050011
(43) Date of publication of application: 19.09.2007
(73) Proprietor: DSM IP Assets B.V., 6411 TE Heerlen (NL)
(72) Inventor: SALONEN, Jukka T., FI-70100 Kuopio (FI); AALTO, Juha-matti, FI-71800 Siilinjärvi (FI); FUENTES, Ricardo, FI-70110 Kuopio (FI); KONTKANEN, Outi, FI-70840 Kuopio (FI); PIRSKANEN, Mia, FI-70100 Kuopio (FI); UIMARI, Pekka, FI-70500 Kuopio (FI)
(74) Representative: Schwander, Kuno
(86) International application number: PCT/FI2005/050486
(87) International publication number: WO 2006/072654

(56) References cited:
- WO-A1-2004/053158
- WO-A1-2004/067771
- WO-A1-2005/003552
- WO-A2-01/57190
- WO-A2-01/62798
- WO-A2-03/062376
- WO-A2-2004/034969
- WO-A2-2004/074514
- WO-A2-2004/084797
- WO-A2-2005/003766
- WO-A2-2006/010514
- JP-A- 2005 198 640
- US-A1- 2002 198 371
- US-A1- 2004 146 890
- US-B1- 6 812 339
- DAS SWAPAN KUMAR; CHU WINSTON; ZHANG ZHENGXIAN; HASSTEDT SANDRA J; ELBEIN STEVEN C: "Calsquestrin 1 (CASQ1) gene polymorphisms under chromosome 1q21 linkage peak are associated with type 2 diabetes in Northern European Caucasians." DIABETES DEC 2004, vol. 53, no. 12, December 2004 (2004-12), pages 3300-3306, XP002369382 ISSN: 0012-1797
- SHIBATA H; HUYNH D P; PULST S M: "A novel protein with RNA-binding motifs interacts with ataxin-2." HUMAN MOLECULAR GENETICS 22 MAY 2000, vol. 9, no. 9, 22 May 2000 (2000-05-22), pages 1303-1313, XP009123262 ISSN: 0964-6906
- DATABASE SNPDB NCBI; 13 November 2003 (2003-11-13), XP007909883 Database accession no. rs10521039
- DATABASE SNPDB NCBI; 13 November 2003 (2003-11-13), XP007909880 Database accession no. rs10500351
- DATABASE snpdb NCBI; 29 September 2001 (2001-09-29), XP007909887 Database accession no. rs2346606
- DATABASE snpdb ncbi; 13 November 2003 (2003-11-13), XP007909881 Database accession no. rs10500352
- DATABASE snpdb ncbi; 1 February 2001 (2001-02-01), XP007909885 Database accession no. rs1922585
- DATABASE snpdb ncbi; 30 June 2000 (2000-06-30), XP007909886 Database accession no. rs225246
- DATABASE snpdb ncbi; 13 November 2003 (2003-11-13), XP007909884 Database accession no. rs10521040
- VAN TILBURG J; VAN HAEFTEN T W; PEARSON P; WIJMENGA C: "Defining the genetic contribution of type 2 diabetes mellitus." JOURNAL OF MEDICAL GENETICS SEP 2001, vol. 38, no. 9, September 2001 (2001-09), pages 569-578, XP007909868 ISSN: 1468-6244
- NADLER S.T. ET AL.: 'The expression of adipogenic genes is decreased in obesity and diabetes mellitus' PROCEEDINGS OF THE NATIONAL ACADEMY OF SCIENCES OF THE UNITED STATES OF AMERICA vol. 97, no. 21, 10 October 2000, pages 11371 - 11376, XP002203186
- PATTI M.E. ET AL.: 'Coordinated reduction of genes of oxidative metabolism in humans with insulin resistance and diabetes: Potential role of PGC1 and NRF1' PROCEEDINGS OF THE NATIONAL ACADEMY OF SCIENCES OF THE UNITED STATES OF AMERICA vol. 100, 2003, pages 8466 - 8471, XP008122553

### SEQUENCE LISTING

<110> Jurilab Ltd
<120> Method and kit for detecting a risk of type 2 diabetes mellitus
<130> xxx
<160> 4685
<170> PatentIn version 3.3
<210> 3214
   <211> 51
   <212> DNA
   <213> Homo sapiens
<400> 3214
   aaagcttacc caggctggta gagaaygggc acggcaagcg tgaagttagg a 51
<210> 3215
   <211> 51
   <212> DNA
   <213> Homo sapiens
<400> 3215
   aacctagtct ttgctgagga caagayatgt tgacaaaccc aggcacaagg a 51
<210> 3216
   <211> 51
   <212> DNA
   <213> Homo sapiens
<400> 3216
   gaatttcttt atggagtggt taatastatg agtccttgct gccaaacagc t 51
<210> 3217
   <211> 51
   <212> DNA
   <213> Homo sapiens
<400> 3217
   cagttagaga aataataaaa taacayctaa aatgggcata tattgataaa a 51

## Description

### BACKGROUND OF THE INVENTION

### Field of the Invention

The present invention relates generally to the field of diagnosis of diabetes mellitus (DM). More particularly, it provides a method of diagnosing or detecting a predisposition or propensity or susceptibility for type 2 diabetes mellitus (T2D). Specifically, the invention is directed to a method that comprises the steps ofproviding a biological sample of the subject to be tested and detecting the presence or absence of one or several genomic single nucleotide polymorphism (SNP) markers in the biological sample. Furthermore, the invention utilises both genetic and phenotypic information as well as information obtained by questionnaires to construct a score that provides the probability of developing T2D.

### Description of Related Art

### Public health significance of T2D

The term diabetes mellitus (DM) (ICD/10 codes E10-E14) describes several syndromes of abnormal carbohydrate metabolism that are characterized by hyperglycemia. It is associated with a relative or absolute impairment in insulin secretion, along with varying degrees of peripheral resistance to the action of insulin. The chronic hyperglycemia of diabetes is associated with long-term damage, dysfunction, and failure of various organs, especially the eyes, kidneys, nerves, heart, and blood vessels (ADA, 2003). T2D is characterized by adult onset insulin resistance and a rise in blood sugar concentration.

In 2000, there were approximately 171 million people, worldwide, with diabetes. The number of people with diabetes will expectedly more than double over the next 25 years, to reach a total of 366 million by 2030 (WHO/IDF, 2004). Most of this increase will occur as a result of a 150% rise in developing countries. This suggests the role of relatively modem environmental or behavioral risk factors such as high caloric intake or sedentary lifestyle. However, ethnic differences in the incidence and prevalence of T2D and the enrichment of T2D in families suggest heritable risk factors to play a major role. In the USA, there are over 15 million diabetics and 15 million people with impaired glucose tolerance. Almost one million Americans become diabetic annually.

The two main contributors to the worldwide increase in prevalence of diabetes are population ageing and urbanization, especially in developing countries, with the consequent increase in the prevalence of obesity (WHO/IDF, 2004). Currently more than I billion adults are overweight - and at least 300 million of them are clinically obese. Current obesity levels range from below 5% in China, Japan and certain African nations, to over 75% in urban Samoa. The prevalence of obesity is 10-25% in Western Europe and 20-27% in the Americas (WHO, 2004).

In 2000, 3.2 million people died from complications associated with diabetes. Diabetes has become one of the major causes of premature illness and death in most countries, mainly through the increased risk of cardiovascular disease (CVD). Diabetes is a leading cause ofblindness, amputation and kidney failure. These complications account for much of the social and financial burden of diabetes (WHO/IDF, 2004).

Because of the chronic nature of T2D, the severity of its complications and the means required to control them, diabetes is a costly disease, not only for the affected individual and his/her family, but also for the health authorities. In the US direct medical and indirect expenditures attributable to diabetes in 2002 were estimated at $132 billion. Direct medical expenditures alone totalled $91.8 billion and comprised $23.2 billion for diabetes care, $24.6 billion for chronic complications attributable to diabetes, and $44.1 billion for excess prevalence of general medical conditions. Attributable indirect expenditures resulting from lost workdays, restricted activity days, mortality, and permanent disability due to diabetes totalled $39.8 billion (ADA, 2003).

### Classification of DM

According to the new etiologic classification of DM, four categories are differentiated: type I diabetes (T1D), type 2 diabetes (T2D), other specific types, and gestational diabetes mellitus (ADA, 2003). In the United States, Canada, and Europe, over 80% of cases of diabetes are due to T2D, 5 to 10% to T1D, and the remainder to other specific causes.

In T1D, formerly known as insulin-dependent (IDDM), the pancreas fails to produce the insulin which is essential for survival. This form develops most frequently in children and adolescents, but is being increasingly diagnosed later in life. T2D, formerly named non-insulin-dependent (NIDDM), results from the body's inability to respond properly to the action of insulin produced by the pancreas. T2D occurs most frequently in adults, but is being noted increasingly in adolescents as well (WHO, 2004).

### Pathophysiology of T2D

The causes of T2D are multi-factorial and include both genetic and environmental elements that affect beta cell function and tissue insulin sensitivity (muscle, liver, adipose tissue, pancreas). Although there is considerable debate as to the relative contributions of beta-cell dysfunction and reduced insulin sensitivity to the pathogenesis of diabetes, it is generally agreed that both of these factors play important roles (Scheen AJ, 2003).

### Insulin resistance

Insulin resistance is probably the first defect in T2D and begins many years before the onset of symptoms or developing a blood glucose level high enough to make the diagnosis (Martin BC et al, 1992). Insulin resistance occurs in the peripheral cells of the body (primarily muscle and fat cells) and the liver. It is caused by genetic factors (see below) as well as environmental factors. The environmental factors include aging, sedentary lifestyle, and central obesity.

Normally, insulin-signalling proceeds by insulin binding to its cell surface receptor protein, which activates the intrinsic tyrosine kinase activity of the receptor (Saltiel AR and Kahn CR, 2001). The activated receptor kinase then phosphorylates protein substrates within the cell on tyrosine residues. These tyrosine phosphorylated insulin receptor substrate proteins act as docking sites, binding other cellular signalling molecules that serve as adapters in the formation of complexes of intracellular signaling proteins. Downstream from these signalling complexes, the various cellular actions of insulin on glucose and lipid metabolism as well as cell growth are stimulated.

Recent research has delineated specific mechanisms whereby excess adiposity can influence the normal cellular actions of insulin and contribute to insulin resistance. Among the factors released by adipose tissue free fatty acids (FFA) are elevated in persons with increased visceral adipose tissue (Boden G, 2001; Shulman GI, 2000). When FFAs are elevated for a prolonged amount of time, they have a direct effect on insulin action in skeletal muscle tissue and liver, reducing the normal responses to insulin to promote glucose uptake and to suppress hepatic glucose output, respectively (Boden G, 2001; Shulman GI, 2000). In both of these tissues, FFA increase cellular levels of acyl-CoA derivatives, which leads to an increase in the activity of cellular signalling molecules termed serine kinases that oppose the normal tyrosine phosphorylation cascade of the insulin receptor (Zick Y, 2001). The increased intracellular lipid accumulation that occurs in obese subjects as ectopic fat-that is, triglyceride stored in the target organs themselves rather than in a benign adipose depot-is another important source of intracellular acyl-CoA molecules that can affect normal insulin signal transduction (Ravussin E and Smith SR, 2002).

Other proteins secreted by adipose tissue, including the important inflammatory mediators interleukin-6 (IL-6) and tumor necrosis factor alpha (TNFa), have adverse effects on energy metabolism and insulin sensitivity in liver and muscle and play key roles in the development of insulin resistance in obesity (Dandona P et al, 2004). Adiponectin is a recently described plasma protein secreted uniquely from adipose tissue. Unlike TNFα and FFA, whose plasma levels are increased in visceral obesity, the levels of adiponectin are reduced in obese subjects (Berg AH et al, 2002). Studies ofthe physiologic role of adiponectin have provided evidence that it enhances insulin action and improves sensitivity as well as having anti-inflammatory protective effects on the vascular endothelium. Adiponectin improves the plasma clearance of FFA, glucose, and triglycerides and suppresses hepatic glucose production (Berg AH et al, 2002). In liver, skeletal muscle, and adipose tissue, the mechanism of action of adiponectin has been shown to involve adenosine monophosphate activated protein kinase (AMP kinase) a signalling kinase implicated in the insulin dependent uptake of glucose into skeletal muscle and also in the cellular mechanism of action of metfonnin (Wu X et al, 2001;Yamauchi T et al, 2002; Tomas E et al, 2002). Also, consistent with the influence of visceral adiposity on insulin resistance and the metabolic syndrome, the regulation of circulating levels of adiponectin appears to be at the level of omental adipose tissue as opposed to the subcutaneous depot (Motoshima H et al, 2002).

### Insulin deficiency

As insulin resistance develops, the beta cells increase insulin production to compensate and maintain the blood glucose level in the narrow range needed for normal body function. If insulin resistance persists or increases over time (usually 3-5 years) the beta cells will begin to fail. When insulin resistance persists but insulin secretion decreases and blood glucose levels begin to rise, true diabetes has developed (Guthrie RA and Guthrie DW, 2004). The pattern of beta cell function loss is an initial defect in acute or first-phase insulin secretion, followed by a decreasing maximal capacity of insulin secretion. Last, a defective steady-state and basal insulin secretion develops, leading to almost complete beta-cell failure requiring insulin treatment. Because ofthe reciprocal relation between insulin secretion and insulin sensitivity, valid representation of beta cell function requires interpretation of insulin responses in the context of the prevailing degree of insulin sensitivity (Scheen AJ, 2004). Evidence of the progressive loss of beta cell function may include altered conversion of proinsulin to insulin, changes in pulsed and oscillatory insulin secretion, and quantitative reductions in insulin release. Potential underlying mechanisms are glucose toxicity, lipotoxicity, poor tolerance of increased secretory demand, and a reduction in beta-cell mass (Buchanan TA, 2003). The roles of glucose and fatty acids in altering the function of various cell types in diabetes, in particular that of the beta cells is summarized in the "glucolipotoxicity" hypothesis (Prentki M et al, 2002). It is suggested that either hyperglycemia alone or elevated circulating FFAs alone should not be so detrimental to a cell for the simple reason that when glucose levels alone are high, glucose is oxidized, and when FFAs alone are high, then they are oxidized instead of glucose. However, when both glucose and FFA levels are high they may progressively alter the function of various cell types. Under this condition, FFA-derived long-chain acyl-CoA esters (FACoAs) are high, and furthermore they cannot be oxidized because glucose-derived malonyl-CoA is also elevated. Malonyl-CoA is a metabolic signalling molecule that regulates lipid partitioning (the relative fluxes of FFA oxidation and esterification) through its inhibitory action on carnitine galmitoyttransferase-1 (CPT1), which catalyzes the rate-limiting step of the mitochondrial β-oxidation of fatty acids. As a result, FACoAs accumulate in the cytoplasm and could, for example, promote beta-cell apoptosis. Indeed, it is now realized that, in the vast majority of T2D cases, there is a decreased beta-cell mass caused by a marked increase in beta-cell apoptosis that outweighs rates of beta-cell mitogenesis and neogenesis. Recent advances have implicated signal transduction via insulin receptor substrate-2 (IRS-2) and downstream via protein tyrosine kinase 2 beta (PTK2B) as critical to the control of beta cell survival (Dickson LM and Rhodes CJ, 2004).

### T2D: a polygenic disease

T2D has a complex mode of inheritance, as corroborated by family studies indicating major roles of both genetic susceptibility and the environment. There was significant familial aggregation of T2DM and related phenotypes. Generally, the sibling of a patient with T2D has a four to six-fold higher risk of developing the disease (30%-40%) than does an unrelated individual (7%) (Florez JC et al, 2003). A strong genetic influence has also been suggested by a greater concordance rate for type 2 diabetes in monozygotic (MZ) compared with dizygotic (DZ) twin pairs. Rates of concordance of T2D are much higher for monozygotic twins as compared to dizygotic twins. It has been reported that 90% of identical twin pairs were concordant for T2D if followed for long enough (Barnett AH et al, 1981). A concordance rate for T2D of 43% in Danish dizygotic twins as compared to 63% in monozygotic twins has also been reported (Poulsen et al, 1999). The heritability of T2D, glucose intolerance and insulin secretion has been estimated to be 60-80 % (Lowe 2001, Lehtovirta et al 2005).

Known monogenic forms of diabetes are classified in two categories: genetic defects of the beta cell and genetic defects in insulin action (ADA, 2003). The diabetes forms associated with monogenetic defects in beta cell function are frequently characterized by onset of hyperglycemia at an early age (generally before age 25 years). They are referred to as maturity-onset diabetes of the young (MODY) and are characterized by impaired insulin secretion with minimal or no defects in insulin action (Herman WH et al, 1994; Clement K et all, 1996; Byrne MM et al, 1996). They are inherited in an autosomal dominant pattern. Abnormalities at three genetic loci on different chromosomes have been identified to date. The most common form is associated with mutations on chromosome 12q in the locus of a hepatic transcription factorreferred to as hepatocyte nuclear factor (HNF)-1α (Vaxillaire M et all,1995; Yamagata K et al, 1996). A second form is associated with mutations in the locus of the glucokinase gene on chromosome 7p and results in a defective glucokinase molecule (Froguel P et al, 1992; Vionnet N et al, 1992). Glucokinase converts glucose to glucose-6-phosphate, the metabolism of which, in turn, stimulates insulin secretion by the beta cell Because of defects in the glucokinase gene, increased plasma levels of glucose are necessary to elicit normal levels of insulin secretion. A third form is associated with a mutation in the HNF-4α gene on chromosome 20q (Bell GI et al, 1991; Yamagata K et al, 1996). HNF-4α is a transcription factor involved in the regulation of the expression of HNF-1α. Point mutations in mitochondrial DNA can cause DM primarily by impairing pancreatic beta cell function (Reardon W et al, 1992; van den Ouwenland JMW et al, 1992; Kadowaki T et al, 1994). There are unusual causes of diabetes that result from genetically determined abnormalities of insulin action. The metabolic abnormalities associated with mutations of the insulin receptor may range from hyperinsulinemia and modest hyperglycemia to severe diabetes (Kahn CR et al, 1976; Taylor SI, 1992).

In most cases, T2D results from a complex interaction of genetic, environmental, and demographic factors. Improved techniques of genetic analysis, especially candidate gene association studies and genome wide linkage analysis (genome wide scan, GWS), have enabled a search for genes that contribute to the development of T2D in the population. No major single genes explaining the development of T2D have been identified. However, studies have demonstrated associations between various metabolic defects underlying the development of type 2 diabetes and polymorphisms in several susceptibility genes (e.g., PPARγ and PGC-1). Although more than a hundred candidate genes have been evaluated for T2D, only a handful have been widely replicated. The association of PPARγ with T2D is widely reproduced (Deeb SS et al, 1998; Hara K et al, 2000; Altshulert D et al, 2000; Mori H et al, 2001), and that of KCNJ1 (Hani EH et al, 1998; Gloyn AL et al, 2001; Gloyn AL et al, 2003), ABCC8 (Inoue H, 1996; Hani EH et al, 1997; Hansen T et al, 1998), GCGR (Hager J et al, 1995; Gough SC et al, 1995), GCK (Chiu KC et al, 1992; McCarthy MI et al, 1994; Takekawa K et al, 1994) and **SLC2A1** (Li SR et al, 1988; Tao T et al, 1995; Pontiroli AE et al, 1996) have now been seen by multiple groups. An example of recent, still uncorroborated, findings is **ARNT** gene, a transcription factor essential for embryonic development (Gunton et al 2005).

Gene-environment interactions have been found between PPARG and birth weight affecting adult insulin sensitivity (Eriksson JG et al, 2002) and between PPARG and dietary fat intake influencing adult BMI (Luan J et al, 2001; Memisoglu A et al, 2003). A gene-gene interaction has also been found between PPARG and FABP4 affecting adult insulin sensitivity and body composition (Damcott CM et al, 2004).

To date more than 30 GWSs have been reported to identify loci for T2D. Linked loci with at least suggestive LOD scores have been observed on every chromosome. Perhaps most striking is the lack of consistently linked loci. Demenais F et al, 2003 applying the genome-search meta-analysis method (GSMA) to 4 published genome-wide scans of T2D from Caucasian populations (GIFT consortium, Finland, Sweden, UK and France) found evidence of susceptibility regions for T2D on chromosomes 1p13.1-q22, 2p22.1-p13.2, 6q21-q24.1, 12q21.1-q24.12, 16p123-q11.2 and 17p11.2-q22, which had modest or non-significant linkage in each individual study. This may serve to illustrate the heterogeneity of human T2D as well as the potential shortcomings of attempting to compare studies using different methodologies.

### Opportunity for population genetics

Developments in GWS and sequencing technology and methods of data analysis render now possible the attempt to identify liability genes in complex, multifactorial traits, and to dissect out with new precision the role of genetic predisposition and environment/life style factors in these disorders. Genetic and environmental effects vary over the life span, and only longitudinal studies in genetically informative data sets permit the study of such effects. A major advantage of population genetics approaches in disease gene discovery over other methodologies is that it will yield diagnostic markers which are valid in humans.

Identification of genes causing the major public health problems such as T2D is now enabled by the following recent advances in molecular biology, population genetics and bioinformatics: 1. the availability of new genotyping platforms that will dramatically lower operating cost and increase throughputs; 2. the application of genome scans using dense marker maps (> 100.000 markers); 3. data analysis using new powerful statistical methods testing for linkage disequilibrium using haplotype sharing analysis, and 4. the recognition that a smaller number of genetic markers than previously thought is sufficient for genome scans in genetically homogeneous populations.

Traditional GWS using microsatellite markers with linkage analyses have not been successful in finding genes causing common diseases. The failure has in part been due to too small a number of genetic markers used in GWS, and in part due to too heterogeneous study populations. With the advancements of the human genome project and genotyping technology, the first dense marker maps have recently become available for mapping the entire human genome. The microarrays used by Jurilab include probes for over 100 000 SNP markers. These SNPs form a marker map covering, for the first time, the entire genome tightly enough for the discovery of the majority of disease genes causing T2D.

### Genetic homogeneity of the East Finland founder population

Finns descend from two human immigration waves occurring about 4,000 and 2,000 years ago, respectively. Both Y-chromosomal haplotypes and mitochondrial sequences show low genetic diversity among Finns compared with other European populations and confirm the long-standing isolation of Finland (Sajantila A et al, 1996). During King Gustaws of Vasa (1523-1560) over 400 years ago, internal migrations created regional subisolates, the late settlements (Peltonen L et al, 1999). The most isolated of these are the East Finns.

The East Finnish population is the most genetically-homogenous population isolate known that is large enough for effective gene discovery program. The reasons for homogeneity are: the young age of the population (fewer generations); the small number of founders; long-term geographical isolation; and population bottlenecks because of wars, famine and fatal disease epidemics.

Owing to the genetic homogeneity of the East Finland population there are fewer mutations in important disease predisposing genes and the affected individuals share similar genetic background. Because of the stronger linkage disequilibrium (LD), fewer SNPs and fewer subjects are needed for GWS than in other populations.

### SUMMARY OF THE INVENTION

The invention is based on a method for estimating susceptibility or predisposition of an individual to T2D comprising the detection of the presence of SNP markers and haplotypes or an alteration in expression of a T2D risk gene set forth in tables 1 through 2, as well as alterations in the polypeptides encoded by the said T2D risk genes. The alterations may be quantitative, qualitative, or both.

The invention is defined in the appended claims.

In one aspect, the invention relates to methods of diagnosing a predisposition to T2D. The methods of diagnosing a predisposition to T2D in an individual include detecting the presence of SNP markers predicting T2D, as well as detecting alterations in expression of genes which are associated with said markers. The alterations in expression can be quantitative, qualitative, or both.

A further object of the invention is a method of identifying the risk of T2D by detecting SNP markers rs 10500351 a biological sample of the subject. The information obtained from this method can be combined with other information concerning an individual, e.g. results from blood measurements, clinical examination and questionnaires. The blood measurements include but are not restricted to the determination of plasma or serum cholesterol and high-density lipoprotein cholesterol. The information to be collected by questionnaire includes information concerning gender, age, family and medical history such as the family history of obesity and diabetes. Clinical information collected by examination includes e.g. information concerning height, weight, hip and waist circumference and other measures of adiposity and obesity.

The methods of the invention allow the accurate diagnosis of T2D at or before disease onset, thus reducing or minimizing the debilitating effects of T2D. The method can be applied in persons who are free of clinical symptoms and signs of T2D, in those who have family history of T2D or obesity or in those who have elevated level or levels of risk factors of T2D or obesity.

Those skilled in the art will readily recognize that the analysis of the nucleotides present in one or several of the SNP markers of this invention in an individual's nucleic acid can be done by any method or technique capable of determining nucleotides present in a polymorphic site. As it is obvious in the art the nucleotides present in SNP markers can be determined from either nucleic acid strand or from both strands.

The major application of the current invention involves prediction of those at higher risk of developing T2D. Diagnostic tests that define genetic factors contributing to T2D might be used together with or independent of the known clinical risk factors to define an individual's risk relative to the general population. Better means for identifying those individuals at risk for T2D should lead to better preventive and treatment regimens, including more aggressive management of the risk factors for T2D such as obesity and of the risk factors for sequelae of T2D such as cigarette smoking, hypercholesterolemia, elevated LDL cholesterol, low HDL cholesterol, elevated BP, obesity, lack of physical activity, and inflammatory components as reflected by increased C-reactive protein levels or other inflammatory markers. Information on genetic risk may be used by physicians to help convince particular patients to adjust life style (e.g. to stop smoking, to reduce caloric intake, to increase exercise). Finally, preventive measures aimed at lowering blood pressure such as reduction of weight, intake of salt and alcohol can be both better motivated to the patients who are at an elevated risk of T2D and selected on the basis of the molecular subdiagnosis of T2D.

### DETAILED DESCRIPTION OF THE INVENTION

### Methods of Therapy

The invention discloses methods to assess the risk of an individual to develop T2D in mammals. A gene test recognizing the T2D risk allele homozygocity or carrier status of T2D susceptibility genes is useful in selecting prophylactic treatment for individuals having a high risk of T2D.

Yet in another embodiment of the invention, a test or a method based on T2D susceptibility gene specific markers (e.g. polymorphic sites, expression or polypeptides) is useful in selecting subjects testing treatments for T2D.

A test or a method of this invention based on T2D susceptibility gene specific markers (e.g. polymorphic sites, expression or polypeptides) is useful in selecting drug therapy for patients who might be at increased risk for adverse effects of drugs affecting T2D susceptibility gene activity.

If the less frequent, i.e. the minor, assumable mutated allele in the T2D susceptibility gene is risk-reducing, and if said mutation is a gene function reducing mutation, one can deduce that the gene function and/or activity would increase the risk of T2D. On that basis, drugs and other therapies such as gene therapies that reduce or inhibit the function or activity of the T2D susceptibility gene or the encoded protein would reduce the risk of the said disease and could be used to both prevent and treat the said disease.

### Representative Target Population

An individual at risk of T2D is an individual who has at least one risk factor, such as family history of T2D or obesity, history of gestational diabetes, previously identified glucose intolerance, obesity, hyperlriglyceridemia, low HDL cholesterol, HT and elevated BP, lack of physical activity, and an at-risk allele or haplotype with one or several T2D risk SNP markers.

In another embodiment of the invention, an individual who is at risk of T2D is an individual who has at least one risk-increasing allele in a T2D risk gene, in which the presence of the polymorphism is indicative of a susceptibility to T2D. The term "gene," as used herein, refers to an entirety containing all regulatory elements located both upstream and downstream as well as within of a polypeptide encoding sequence of a gene and entire transcribed region of a gene including 5' and 3' untranslated regions of mRNA and the entire polypeptide encoding sequence including all exon and intron sequences (also alternatively spliced exons and introns) of a gene.

### Assessment for At-Risk Alleles and At-Risk Haplotypes

The genetic markers are particular "alleles" at "polymorphic sites" associated with T2D. A nucleotide position in genome at which more than one sequence is possible in a population, is referred to herein as a "polymorphic site". Where a polymorphic site is a single nucleotide in length, the site is referred to as a SNP. For example, if at a particular chromosomal location, one member of a population has an adenine and another member of the population has a thymine at the same position, then this position is a polymorphic site, and, more specifically, the polymorphic site is a SNP. Polymorphic sites may be several nucleotides in length due to insertions, deletions, conversions or translocations. Each version of the sequence with respect to the polymorphic site is referred to herein as an "allele" of the polymorphic site. Thus, in the previous example, the SNP allows for both an adenine allele and a thymine allele.

Typically, a reference nucleotide sequence is referred to for a particular gene e.g. in NCBI databases (www.ncbi.nlm.nih.gov). Alleles that differ from the reference are referred to as "variant" alleles. The polypeptide encoded by the reference nucleotide sequence is the "reference" polypeptide with a particular reference amino acid sequence, and polypeptides encoded by variant alleles are referred to as "variant" polypeptides with variant amino acid sequences.

Nucleotide sequence variants can result in changes affecting properties of a polypeptide. These sequence differences, when compared to a reference nucleotide sequence, include insertions, deletions, conversions and substitutions: e.g. an insertion, a deletion or a conversion may result in a frame shift generating an altered polypeptide; a substitution of at least one nucleotide may result in a premature stop codon, amino acid change or abnormal mRNA splicing; the deletion of several nucleotides, resulting in a deletion of one or more amino acids encoded by the nucleotides; the insertion of several nucleotides, such as by unequal recombination or gene conversion, resulting in an interruption of the coding sequence of a reading frame; duplication of all or a part of a sequence,; transposition; or a rearrangement of a nucleotide sequence, as described in detail above. Such sequence changes alter the polypeptide encoded by a T2D susceptibility gene. For example, a nucleotide change resulting in a change in polypeptide sequence can alter the physiological properties of a polypeptide dramatically by resulting in altered activity, distribution and stability or otherwise affect on properties of a polypeptide.

Alternatively, nucleotide sequence variants can result in changes affecting transcription of a gene or translation of its mRNA. A polymorphic site located in a regulatory region of a gene may result in altered transcription of a gene e.g. due to altered tissue specificity, altered transcription rate or altered response to transcription factors. A polymorphic site located in a region corresponding to the mRNA of a gene may result in altered translation of the mRNA e.g. by inducing stable secondary structures to the mRNA and affecting the stability of the mRNA. Such sequence changes may alter the expression of a T2D susceptibility gene.

A "haplotype," as described herein, refers to any combination of genetic markers ("alleles"). A haplotype can comprise two or more alleles and the length of a genome region comprising a haplotype may vary from few hundred bases up to hundreds of kilobases. As it is recognized by those skilled in the art the same haplotype can be described differently by determining the haplotype defining alleles from different nucleic acid strands. E.g. the haplotype CAC defined by the SNP markers rs2400503 (C/T), rs10515605 (A/G), and rs7709159 (C/T) is the same as the haplotype GTG in which the SNP markers rs2400503 (C/T), rs 10515605 (A/G), and rs7709159 (C/T) are determined from the complementary strand or haplotype GAC in which the SNP marker rs2400503 (C/T) is determined from the complementary strand. The haplotypes described herein are found more frequently in individuals with T2D risk than in individuals without T2D risk. Therefore, these haplotypes have predictive value for detecting T2D risk or a susceptibility to T2D in an individual. Therefore, detecting haplotypes can be accomplished by methods known in the art for detecting sequences at polymorphic sites.

It is understood that the T2D associated at-risk alleles and at-risk haplotypes described in this invention may be associated with other "polymorphic sites" located in T2D associated genes of this invention. These other T2D associated polymorphic sites may be either equally useful as genetic markers or even more useful as causative variations explaining the observed association of at-risk alleles and at-risk haplotypes of this invention to T2D.

In certain methods described herein, an individual who is at risk for T2D is an individual in whom an at-risk allele or an at-risk haplotype is identified. In one embodiment, the at-risk allele or the at-risk haplotype is one that confers a significant risk of death. In one embodiment, significance associated with an allele or a haplotype is measured by an odds ratio. In a further embodiment, the significance is measured by a percentage. In one embodiment, a significant risk is measured as odds ratio of 0.8 or less or at least about 1.2, including by not limited to: 0.1, 0.2, 0.3, 0.4, 0.5, 0.6, 0.7, 0.8, 1.2, 1.3, 1.4, 1.5, 1.6, 1.7, 1.8, 1.9, 2.0, 2.5, 3.0, 4.0, 5.0, 10.0, 15.0, 20.0, 25.0, 30.0 and 40.0. In a further embodiment, a significant increase or reduction in risk is at least about 20%, including but not limited to about 25%, 30%, 35%, 40%, 45%, 50%, 55%, 60%, 65%, 70%, 75%, 80%, 85%, 90%, 95% and 98%. In a further embodiment, a significant increase in risk is at least about 50%. It is understood however, that identifying whether a risk is medically significant may also depend on a variety of factors, including the specific disease, the allele or the haplotype, and often, environmental factors.

An at-risk haplotype in, or comprising portions of, the T2D risk gene, is one where the haplotype is more frequently present in an individual at risk for T2D (affected), compared to the frequency of its presence in a healthy individual (control), and wherein the presence of the haplotype is indicative of T2D risk or susceptibility to T2D.

In a preferred embodiment, the method comprises assessing in an individual the presence or frequency of SNP marker rs 10500351 in, comprising portions of, a T2D risk gene, wherein an excess or higher frequency of the SNP marker compared to a healthy control individual is indicative that the individual has T2D risk, or is susceptible to T2D. The presence of the haplotype is indicative of T2D risk, or a susceptibility to T2D, and therefore is indicative of an individual who falls within a target population for the treatment methods described herein.

### Diagnostic Assays

The markers, probes, primers and antibodies described herein can be used in methods and kits used for risk assessment, diagnosis or prognosis of T2D or a disease or condition associated with T2D in a subject.

According to the invention, the diagnosis of risk or susceptibility to T2D (or diagnosis of or susceptibility to a disease or condition associated with T2D), is made by detecting SNP rs 10500351.

According to the disclosure, the diagnosis of risk or susceptibility to T2D (or diagnosis of or susceptibility to a disease or condition associated with T2D), is made by detecting one or several of polymorphic sites which are associated with at-risk alleles or/and at-risk haplotypes described in this invention in the subject's nucleic acid. Diagnostically the most useful polymorphic sites are those altering the polypeptide structure of a T2D associated gene due to a frame shift; due to a premature stop codon, due to an amino acid change or due to abnormal mRNA splicing. Nucleotide changes resulting in a change in polypeptide sequence in many cases alter the physiological properties of a polypeptide by resulting in altered activity, distribution and stability or otherwise affect on properties of a polypeptide. Other diagnostically useful polymorphic sites are those affecting transcription of a T2D associated gene or translation of it's mRNA due to altered tissue specificity, due to altered transcription rate, due to altered response to physiological status, due to altered translation efficiency of the mRNA and due to altered stability of the mRNA. The presence of nucleotide sequence variants altering the polypeptide structure of T2D associated genes or altering the expression of T2D associated genes is diagnostic for susceptibility to T2D.

For diagnostic applications, there may be polymorphisms informative for prediction of disease risk, which are in linkage disequilibrium with the functional polymorphism. Such a functional polymorphism may alter splicing sites, affect the stability or transport of mRNA, or otherwise affect the transcription or translation of the nucleic acid. The presence of nucleotide sequence variants associated with a functional polymorphism is diagnostic for susceptibility to T2D. While we have genotyped and included a limited number of example SNP markers in the experimental section, any functional, regulatory or other mutation or alteration described above in any of the T2D risk genes identified herein is expected to predict the risk of T2D.

In diagnostic assays determination of the nucleotides present in one or several of the T2D associated SNP markers of this disclosure as well as polymorphic sites associated with T2D associated SNP markers of this disclosure in an individual's nucleic acid can be done by any method or technique which can accurately determine nucleotides present in a polymorphic site. Numerous suitable methods have been described in the art (see e.g. KwokP-Y, 2001; Syvänen A-C, 2001), these methods include, but are not limited to, hybridization assays, ligation assays, primer extension assays, enzymatic cleavage assays, chemical cleavage assays and any combinations of these assays. The assays may or may not include PCR, solid phase step, a microarray, modified oligonucleotides, labeled probes or labeled nucleotides and the assay may be multiplex or singleplex. As it is obvious in the art the nucleotides present in a polymorphic site can be determined from either nucleic acid strand or from both strands.

In another embodiment of the disclosure, diagnosis of a susceptibility to T2D can be assessed by examining transcription of one or several T2D associated genes. Alterations in transcription can be assessed by a variety of methods described in the art, including e.g. hybridization methods, enzymatic cleavage assays, RT-PCR assays and microanays. A test sample from an individual is collected and the alterations in the transcription of T2D associated genes are assessed from the RNA present in the sample. Altered transcription is diagnostic for a susceptibility to T2D.

In another embodiment ofthe disclosure, diagnosis ofa susceptibility to T2D can also be made by examining expression and/or structure and/or function of a T2D susceptibility polypeptide. A test sample from an individual is assessed for the presence of an alteration in the expression and/or an alteration in structure and/or function of the polypeptide encoded by a T2D risk gene, or for the presence of a particular polypeptide variant (e.g., an isoform) encoded by a T2D risk gene. An alteration in expression of a polypeptide encoded by a T2D risk gene can be, for example, quantitative (an alteration in the quantity of the expressed polypeptide, i.e., the amount of polypeptide produced) or qualitative (an alteration in the structure and/or function of a polypeptide encoded by a T2D risk gene, i.e. expression of a mutant T2D susceptibility polypeptide or of a different splicing variant or isoform). In a preferred embodiment, detecting a particular splicing variant encoded by a T2D risk gene or a particular pattern of splicing variants makes diagnosis of the disease or condition associated with T2D or a susceptibility to a disease or condition associated with T2D.

Alterations in expression and/or structure and/or function of a T2D susceptibility polypeptide can be determined by various methods known in the art e.g. by assays based on chromatography, spectroscopy, colorimetry, electrophoresis, isoelectric focusing, specific cleavage, immunologic techniques and measurement of biological activity as well as combinations of different assays. An "alteration" in the polypeptide expression or composition, as used herein, refers to an alteration in expression or composition in a test sample, as compared with the expression or composition in a control sample and an alteration can be assessed either directly from the T2D susceptibility polypeptide or it's fragment or from substrates and reaction products of said polypeptide. A control sample is a sample that corresponds to the test sample (e.g., is from the same type of cells), and is from an individual who is not affected by T2D. An alteration in the expression or composition of a polypeptide encoded by a T2D susceptibility gene of the disclosure in the test sample, as compared with the control sample, is indicative of a susceptibility to T2D.

Western blotting analysis, using an antibody as described above that specifically binds to a polypeptide encoded by a mutant T2D risk gene or an antibody that specifically binds to a polypeptide encoded by a non-mutant gene, or an antibody that specifically binds to a particular splicing variant encoded by a T2D risk gene can be used to identify the presence or absence in a test sample of a particular polypeptide encoded by a polymorphic or mutant T2D risk gene. The presence of a polypeptide encoded by a polymorphic or mutant gene, or the absence of a polypeptide encoded by a non-polymorphic or non-mutant gene, is diagnostic for a susceptibility to T2D, as is the presence (or absence) of particular splicing variants encoded by a T2D risk gene.

In one embodiment of this method, the level or amount of a polypeptide encoded by a T2D risk gene in a test sample is compared with the level or amount of the same polypeptide encoded by the same T2D risk gene in a control sample. A level or amount of the polypeptide in the test sample that is higher or lower than the level or amount of the polypeptide in the control sample, such that the difference is statistically significant, is indicative of an alteration in the expression of the polypeptide encoded by a T2D risk gene, and is diagnostic for a susceptibility to T2D. Alternatively, the composition of the polypeptide encoded by a T2D risk gene in a test sample is compared with the composition of the polypeptide encoded by a T2D risk gene in a control sample (e.g., the presence of different splicing variants). A difference in the composition of the polypeptide in the test sample, as compared with the composition of the polypeptide in the control sample, is diagnostic for a susceptibility to T2D. In another embodiment, both the level or amount and the composition of the polypeptide can be assessed in the test sample and in the control sample. A difference in the amount or level of the polypeptide in the test sample, compared to the control sample; a difference in composition in the test sample, compared to the control sample; or both a difference in the amount or level, and a difference in the composition, is indicative of a susceptibility to T2D.

In another embodiment, assessment of the splicing variant or isoform(s) of a polypeptide encoded by a polymorphic or mutant T2D risk gene can be performed. The assessment can be performed directly (e.g., by examining the polypeptide itself), or indirectly (e.g., by examining the mRNA encoding the polypeptide, such as through mRNA profiling). For example, probes or primers as described herein can be used to determine which splicing variants or isoforms are encoded by a T2D risk gene mRNA, using standard methods.

The presence in a test sample of a particular splicing variant(s) or isoform(s) associated with T2D or risk of T2D, or the absence in a test sample of a particular splicing variant(s) or isoform(s) not associated with T2D or risk of T2D, is diagnostic for a disease or condition associated with a T2D risk gene or a susceptibility to a disease or condition associated with a T2D risk gene. Similarly, the absence in a test sample of a particular splicing variant(s) or isoform(s) associated with T2D or risk of T2D, or the presence in a test sample of a particular splicing variant(s) or isoform(s) not associated with T2D or risk of T2D, is diagnostic for the absence of disease or condition associated with a T2D risk gene or a susceptibility to a disease or condition associated with a T2D risk gene.

The disclosure pertains to a method for the diagnosis and identification of susceptibility to T2D in an individual, by assessing markers present in at-risk alleles or at-risk haplotypes of T2D risk genes. In one embodiment, the at-risk allele or the at-risk haplotype is an allele or a haplotype for which the presence of the allele or the haplotype increases the risk of T2D significantly. Although it is to be understood that identifying whether a risk is significant may depend on a variety of factors, including the specific disease, the haplotype, and often, environmental factors, the significance may be measured by an odds ratio or a percentage. In a further embodiment, the significance is measured by a percentage. In one embodiment, a significant risk is measured as an odds ratio of 0.8 or less or at least about 1.2, including by not limited to: 0.1, 0.2, 0.3, 0.4, 0.5, 0.6, 0.7, 0.8, 1.2,1.3,1.4, 1.5, 1.6, 1.7, 1.8, 1.9, 2.0, 2.5, 3.0, 4.0, 5.0, 10.0, 15.0, 20.0, 25.0, 30.0 and 40.0. In a further embodiment, an odds ratio of at least 1.2 is significant. In a further embodiment, an odds ratio of at least about 1.5 is significant. In a further embodiment, a significant increase or decrease in risk is at least about 1.7. In a further embodiment, a significant increase in risk is at least about 20%, including but not limited to about 25%, 30%, 35%, 40%, 45%, 50%, 55%, 60%, 65%, 70%, 75%, 80%, 85%, 90%, 95% and 98%. In a further embodiment, a significant increase orreduction in risk is at least about 50%. It is understood however, that identifying whether a risk is medically significant may also depend on a variety of factors, including the specific disease, the allele or the haplotype, and often, environmental factors.

The invention also pertains to methods of diagnosing risk or a susceptibility to T2D in an individual, comprising screening for an at-risk haplotype in a T2D risk gene that is more frequently present in an individual susceptible to T2D (affected), compared to the frequency of its presence in a healthy individual (control), wherein the presence of the haplotype is indicative of risk or susceptibility to T2D.

Kits (e.g., reagent kits) useful in the methods of diagnosis comprise components useful in any of the methods described herein, including for example, PCR primers, hybridization probes or primers as described herein (e.g., labeled probes or primers), reagents for genotyping SNP markers, reagents for detection of labeled molecules, restriction enzymes (e.g., for RFLP analysis), allele-specific oligonucleotides, DNA polymerases, RNA polymerases, marker enzymes, antibodies which bind to altered or to non-altered (native) T2D susceptibility polypeptide, means for amplification of nucleic acids comprising one or several T2D risk genes, or means for analyzing the nucleic acid sequence of one or several T2D risk genes or for analyzing the amino acid sequence of one or several T2D susceptibility polypeptides, etc. In one embodiment of the disclosure, a kit for diagnosing susceptibility to T2Dcan comprise primers for nucleic acid amplification of fragments from a T2D risk gene comprising markers defining an at-risk haplotype that is more frequently present in an individual susceptible to T2D. The primers can be designed using portions of the nucleic acid sequence flanking SNPs that are indicative of T2D.

This invention is based on the principle that one or a small number of genotype analyses are performed, and the mutations to be typed are selected on the basis of their ability to predict T2D. For this reason any method to genotype mutations in a genomic DNA sample can be used. If non-parallel methods such as real-time PCR are used, the genotype analyses are done in a row. The PCR reactions may be multiplexed or carried out separately in a row or in parallel aliquots.

Thus, the detection method of the invention may further comprise a step of combining information concerning age, gender, smoking status, physical activity, waist-to-hip circumference ratio (cm/cm), the subject family history of T2D or obesity, history of gestational diabetes, previously identified glucose intolerance, obesity, hypertriglyceridemia, low HDL cholesterol, HT and elevated BP. The detection method of the invention may also further comprise a step determining blood, serum or plasma glucose, total cholesterol, HDL cholesterol, LDL cholesterol, triglyceride, apolipoprotein B and AI, fibrinogen, ferritin, transferrin receptor, C-reactive protein, serum or plasma insulin concentration.

The score that predicts the probability of T2D may be calculated e.g. using a multivariate failure time model or a logistic regression equation. The results from the further steps of the method as described above render possible a step of calculating the probability of T2D using a logistic regression equation as follows.

Probability of T2D = 1/[1 + e (-(-a + ∑(bi*Xi))], where e is Napier's constant, Xi are variables related to the T2D, bi are coefficients of these variables in the logistic function, and a is the constant term in the logistic function, and wherein a and bi are preferably determined in the population in which the method is to be used, and Xi are preferably selected among the variables that have been measured in the population in which the method is to be used. Preferable values for bᵢ are between -20 and 20; and for i between 0 (none) and 100,000. A negative coefficient bᵢ implies that the marker is risk-reducing and a positive that the marker is risk-increasing. Xi are binary variables that can have values or are coded as 0 (zero) or 1 (one) such as SNP markers. The model may additionally include any interaction (product) or terms of any variables Xi, e.g. biXi. An algorithm is developed for combining the information to yield a simple prediction of T2D as percentage of risk in one year, two years, five years, 10 years or 20 years. Alternative statistical models are failure-time models such as the Cox's proportional hazards' model, other iterative models and neural networking models.

The test can be applied to test the risk of developing a T2D in both healthy persons, as a screening or predisposition test and high-risk persons (who have e.g. family history of T2D or history of gestational diabetes or previous glucose intolerance or obesity or any combination of these or elevated level of any other T2D risk factor).

The method can be used in the prediction and early diagnosis of T2D in adult persons, stratification and selection ofsubjects in clinical trials, stratification and selection of persons for intensified preventive and curative interventions. The aim is to reduce the cost of clinical drug trials and health care.

### EXPERIMENTAL SECTION

### Study populations: The KIHD and NSP cohorts

**KIHD:** The Kuopio Ischaemic Heart Disease Risk Factor Study (KIHD) is an ongoing prospective population-based cohort study, which was designed to investigate genetic and other risk factors for cardiovascular and metabolic diseases and related outcomes in the East Finland founder population, known for its genetic homogeneity and high occurrence of CHD (Salonen JT 1988, Salonen JT et al 1998, 1999, Tuomainen T-P et al 1999).

The study population was a random age-stratified sample of men living in Eastern Finland who were 42, 48, 54 or 60 years old at baseline examinations in 1984-1989. A total of 2682 men were examined during 1984-89. The male cohort was complemented by a random population sample of 920 women, first examined during 1998-2001, at the time of the 11-year follow up of the male cohort. The recruitment and examination ofthe subjects has been described previously in detail (Salonen JT, 1988). The University of Kuopio and University Hospital Ethics Committee approved the study. All participants gave their written informed consent.

**NSP:** In addition to the existing KIHD cohort, a large population cohort was examined in 2003 in Eastern Finland. This "North Savo Project" (NSP) includes the collection of disease, family, drug response and contact information. By October 2004, 17,100 participants have been surveyed. The study consists of the identification ofprobands with T2D, the collection of blood for DNA extraction and consent to use it. In the second phase, patients with T2D and T2D-free controls were examined.

### Definition of cases and controls

The subjects of the present study were participants in either the KIHD or the NSP. In the KIHD, fasting blood glucose was measured using a glucose dehydrogenase method after precipitation of proteins by trichloroacetic acid. Serum insulin was determined with a Novo Biolabs radioimmunoassay kit (Novo Nordisk). In the NSP, prevalent diabetes was assessed by medication review and fasting blood glucose level, obtained from whole blood samples after at least 12 hours of overnight fasting and measured with the glucose dehydrogenase method after precipitation of the proteins with trichloroacetic acid (Granutest 100, Merck). A person was considered diabetic if he/she currentlyused diet or took medication to control blood glucose or if he/she had a fasting blood glucose level of ≥ 6.7 mmol/L (120 mg/dL).

The cases had T2D and family history or T2D. All T2D cases (probands) had at least one additional affected relative, who was parent, sibling or offspring of the proband. Most of them had more than one additional affected family member. The controls had neither T2D nor family history of T2D. During the first phase ofthe study 93 cases and 157 controls were analyzed (first analysis) using the technology access version or commercial version of Affymetrix GeneChip® Human Mapping 100k assay. In the second phase of the study 30 cases and 29 controls were added, so that a second analysis was based on a set of 123 and 186 controls using either the technology access version or a commercial version of Affymetrix GeneChip® Human Mapping 100k assay. The third analysis was based on 80 cases and 30 controls (who were included in the second analysis) using only the commercial version of Affymetrix GeneChip® Human Mapping 100k assay. Combined results from the analyses are presented in the results section.

### Other phenotypic measurements

Age and tobacco smoking were recorded on a self-administered questionnaire checked by an interviewer. HDL fractions were separated from fresh serum by combined ultracentrifugation and precipitation. The cholesterol contents of lipoprotein fractions and serum triglycerides were measured enzymatically. Both systolic and diastolic BPs were measured in the morning by a nurse with a random-zero mercury sphygmomanometer. The measuring protocol included three measurements in supine, one in standing and two in sitting position with 5-minutes intervals. The mean of all six measurements were used as SBP and DBP (Salonen JT et al, 1998). Body mass index (BMI) was computed as the ratio of weight to the square of height (kg/m²). Waist-to-hip ratio (WHR) was calculated as the ratio of waist circumference (average of one measure taken after inspiration and one taken after expiration at the midpoint between the lowest rib and the iliac crest) to hip circumference (measured at the level of the trochanter major).

### Genomic DNA isolation and quality testing

High molecular weight genomic DNA samples were extracted from frozen venous whole blood using standard methods and dissolved in standard TE buffer. The quantity and purity of each DNA sample was evaluated by measuring the absorbance at 260 and 280 nm and integrity of isolated DNA samples was evaluated with 0,9% agarose gel electrophoresis and Ethidiumbromide staining. A sample was qualified for genome wide scan (GWS) analysis if A260/A280 ratio was ≥1.7 and/or average size of isolated DNA was over 20 kb in agarose gel electrophoresis. Before GWS analysis samples were diluted to concentration of 50 ng/µl in reduced EDTA TE buffer (TEKnova).

### Genome-Wide Scan

Genotyping of SNP markers was performed by using either the technology access version or a commercial version of Affymetrix GeneChip® Human Mapping 100k assay. The assay consisted of two arrays, Xba and Hind, which were used to genotype over 100,000 SNP markers from each DNA sample. The assays were performed according to the instructions provided by the manufacturer. A total of 250 ng of genomic DNA was used for each individual assay. DNA sample was digested with either Xba I or Hind III enzyme (New England Biolabs, NEB) in the mixture ofNE Buffer 2 (1 x; NEB), bovine serum albumin (1 x; NEB), and either Xba I or Hind III (0,5 U/ µl; NEB) for 2h at +37°C followed by enzyme inactivation for 20 min at +70C. Xba I or Hind III adapters were then ligated to the digested DNA samples by adding Xba or Hind III adapter (0,25 µM, Affymetrix), T4 DNA ligase buffer (1 x; NEB), and T4 DNA ligase (250 U; NEB). Ligation reactions were allowed to proceed for 2h at +16°C followed by 20 min incubation at +70°C. Each ligated DNA sample was diluted with 75 µl of molecular biology-grade water (BioWhittaker Molecular Applications/Cambrex).

Diluted ligated DNA samples were subjected to three to four identical 100 µl volume polymerase chain reactions (PCR) by implementing a 10 µl aliquot of DNA sample with Pfx Amplification Buffer (1 x; Invitrogen), PCR Enhancer (1 x; Invitrogen), MugsO4 (1 mM; Invitrogen), dNTP (300 µM each; Takara), PCR primer (1 µM; Affymetrix), and Pfx Polymerase (0,05 U/µl; Invitrogen). The PCR was allowed to proceed for 3 min at +94°C, followed by 30 cycles of 15 sec at +94°C, 30 sec at +60°C, 60 sec at +68°C, and finally for the final extension for 7 min at +68°C. The performance of the PCR was checked by standard 2% agarose gel electrophoresis in 1 x TBE buffer for 1h at 120V.

PCR products were purified according to Affymetrix manual using MinElute 96 UF PCR Purification kit (Qiagen) by combining all three to four PCR products of an individual sample into same purification reaction. The purified PCR products were eluted with 40 µl of EB buffer (Qiagen), and the yields of the products were measured at the absorbance 260 nm. A total of 40 µg of each PCR product was then subjected to fragmentation reaction consisting of 0,2 U of fragmentation reagent (Affymetrix) in 1x Fragmentation Buffer. Fragmentation reaction was allowed to proceed for 35 min at +37°C followed by 15 min incubation at +95°C for enzyme inactivation. Completeness of fragmentation was checked by running an aliquot of each fragmented PCR product in 4% TBE gel (4% NuSieve 3:1 Plus Agarose; BMA Reliant precast) for 30-45 min at 120V.

Fragmented PCR products were then labelled using 1 x Terminal Deoxinucleotidyl Transferase (TdT) buffer (Affymetrix), GeneChip DNA Labeling Reagent (0,214 mM; Affymetrix), and TdT (1,5 U/µl; Affymetrix) for 2h at +37°C followed by 15 min at +95°C. Labeled DNA samples were combined with hybridization buffer consisting of 0,056 M MES solution (Sigma), 5% DMSO (Sigma), 2,5 x Denhardt's solution (Sigma), 5,77 mM EDTA (Ambion), 0,115 mg/ml Herring Sperm DNA (Promega), 1 x Oligonucleotide Control reagent (Affymetrix), 11,5 µg/ml Human Cot-1 (Invitrogen), 0,0115% Tween-20 (Pierce), and 2,69 M Tetramethyl Ammonium Chloride (Sigma). DNA-hybridization buffer mix was denatured for 10 min at +95°C, cooled on ice for 10 sec and incubated for 2 min at +48°C prior to hybridization onto corresponding Xba or Hind GeneChip® array. Hybridization was completed at +48°C for 16-18 h at 60 rpm in an Affymetrix GeneChip Hybridization Oven. Following hybridization, the arrays were stained and washed in GeneChip Fluidics Station 450 according to fluidics station protocol Mapping 10Kv1_450 (technology access version arrays) or Mapping 100Kv1_ 450 (commercial version arrays) as recommended by the manufacturer. Arrays were scanned with GeneChip 3000 Scanner, and the genotype calls for each of the SNP markers on the array were generated using Affymetrix Genotyping Tools (GTT) software (technology access version arrays, confidence score in SNP calling algorithm was adjusted to 0.20) or using GeneChip DNA Analysis Software 2.0 (GDAS) (commercial version arrays) with standard settings provided by the manufacturer.

### Initial SNP selection for statistical analysis

Prior to the statistical analysis, SNP quality was assessed on the basis of three values: the call rate (CR), minor allele frequency (MAF), and Hardy-Weinberg equilibrium (H-W). The CR is the proportion of samples genotyped successfully. It does not take into account whether the genotypes are correct or not. The call rate was calculated as: CR = number of samples with successful genotype call / total number of samples. The MAF is the frequency of the allele that is less frequent in the study sample. MAF was calculated as: MAF = min(p , q), where p is frequency of the SNP allele 'A' and q is frequency of the SNP allele 'B'; p = (number of samples with "AA"-genotype + 0.5 *number of samples with "AB"-genotype) / total number of samples with successful genotype call; q = 1 - p. SNPs that are homozygous (MAF=0) cannot be used in genetic analysis and were thus discarded. H-W equilibrium is tested for controls. The test is based on the standard Chi-square test of goodness of fit. The observed genotype distribution is compared with the expected genotype distribution under H-W equilibrium. For two alleles this distribution is p2, 2pq, and q2 for genotypes 'AA', 'AB' and 'BB', respectively. If the SNP is not in H-W equilibrium it can be due to genotyping error or some unknown population dynamics (e.g. random drift, selection).

Based on the control group, only the SNPs that had CR > 50%, MAF > 1%, and were in H-W equilibrium (Chi-square test statistic < 23.93) were used in the statistical analysis. A total of 100,848 SNPs fulfilled the above criteria and were included in the statistical analysis.

### Statistical Methods

### Single SNP analysis

Differences in allele distributions between cases and controls were screened for all SNPs. The screening was carried out using the standard Chi-square independence test with I df (allele distribution, 2x2 table). SNPs that gave a P-value less than 0.005 (Chi-square distribution with 1 df of 7.88 or more) were considered as statistically significant and is reported in the table for rs 10500351. Odds ratio was calculated as ad/bc, where a is the number of minor alleles in cases, b is the number of major alleles in cases, c is the number of minor allele in controls, and d is the number of major alleles in controls. Minor allele was defined as the allele for a given SNP that has smaller frequency than the other allele in the control group.

### Haplotype analysis

The data set was analyzed with a haplotype pattern mining algorithm with HPM software (Toivonen HT et al, 2000). For HPM software, genotypes must be phase known to determine which alleles come from the mother and which from the father. Without family data, phases must be estimated based on population data. We used HaploRec-program (Eronen L et al, 2004) to estimate the phases. HPM is very fast and can handle a large number of SNPs in a single run

For phase-known data HPM finds all haplotype patterns that are in concordance with the phase configuration. The length of the haplotype patterns can vary. As an example, if there are four SNPs and an individual has alleles A T for SNP1, C C for SNP2, C G for SNP3, and A C for SNP4, then HPM considers haplotype patterns that are in concordance with the estimated phase (done by HaploRec). If the estimated phase is ACGA (from the mother/father) and TCCC (from the father/mother) then HPM considers only two patterns (of length 4 SNPs): ACGA and TCCC.

A SNP is scored based on the number of times it is included in a haplotype pattern that differs between cases and controls (a threshold Chi-square value can be selected by the user). Significance of the score values is tested based on permutation tests.

Several parameters can be modified in the HPM program including the Chi-square threshold value (-x), the maximum haplotype pattern length (-1), the maximum number of wildcards that can be included in a haplotype pattern (-w), and the number of permutation tests in order to estimate the P-value (-p). Wildcards allow gaps in haplotypes. HaploRec+ HPM was run with the following parameter settings: haplotype analysis with 5 SNPs (-x9 -15 -w1 -p10000) and haplotype analysis with 8 SNPs (-x9 -18 -w1 - p10000). HaploRec+HPM was based on the order of the SNP given in dbSNP124. Based on 10,000 replicates (-p 10000) SNPs that gave a P-value less than 0.005 were considered as statistically significant and reported in table 2.

Definition of terms used in the haplotype analysis results

The term "haplotype genomic region" or "haplotype region" refers to a genomic region that has been found significant in the haplotype analysis (HPM or similar statistical method/program). The haplotype region is defined as 100Kbp up/downstream from the physical position of the first/last SNP that was included in the statistical analysis (haplotype analysis) and was found statistically significant. This region is given in base pairs based on the given genome build e.g. SNP physical position (base pair position) according to NCBI Human Genome Build 35.

The term "haplotype" as described herein, refers to any combination of alleles e.g. C A G G that is found in the given genetic markers e.g rs1418754, rs2797573, rs787663, rs 10509650. A defined haplotype gives the name of the genetic markers (dbSNP rs-id for the SNPs) and the alleles. As it is recognized by those skilled in the art, the same haplotype can be described differently by determining alleles from different strands e.g. the haplotype rs1418754, rs2797573, rs787663, rs10509650 (C A G G) is the same as haplotype rs 1418754, rs2797573, rs787663, rs 10509650 (G T C C) in which the alleles are determined from the other strand, or haplotype rs1418754, rs2797573, rs787663, rs10509650 (G A G G), in which the first allele is determined from the other strand.

The haplotypes described herein, e.g. having markers such as those shown in table 2, are found more frequently in individuals with T2D than in individuals without T2D. Therefore, these haplotypes have predictive value for detecting T2D or a susceptibility to T2D in an individual. Therefore, detecting haplotypes can be accomplished by methods known in the art for detecting sequences at polymorphic sites.

It is understood that the T2D associated at-risk alleles and at-risk haplotypes described in this invention may be associated with other "polymorphic sites" located in T2D associated genes of this invention. These other T2D associated polymorphic sites may be either equally useful as genetic markers or even more useful as causative variations explaining the observed association of at-risk alleles and at-risk haplotypes of this invention to T2D.

### Results

In table 1 are summarized the characteristics of the SNP marker rs10500351 with the strongest association with T2D in the individual marker analysis combined from both the first and second genome scans. SNP identification number according to NCBI dbSNP database build 124. SNP physical position according to NCBI Human Genome Build 35.1. Gene locus as reported by NCBI dbSNP database build 124. SNP flanking sequence provided by Affymetrix "csv" commercial Human Mapping 100K array annotation files. The genes positioned within 100Kbp up/downstream from the physical position of the SNPs are provided based on the NCBI Human Genome Build 35.1. 2.

In table 2 are summarized the characteristics of the haplotype genomic regions with the strongest association with T2D in the haplotype sharing analysis with 8 SNPs (HaploRec + HPM) from the analysis of the second genome scan.

### Implications and Conclusions

We have found 4685 SNP markers associated with T2D in a population-based set of familial cases and healthy controls without family history. Of these, 1637 were identified in the analysis of individual SNPs and 3751 in the haplotype sharing analysis. Of the 4685 markers, 703 predicted T2D in both types of statistical analysis. Of the 4685 SNP markers associated with the risk of T2D 2407 SNP markers were intragenic.

The results of the point wise and haplotype analyses identified a total of 3048 genes associated with T2D, of which 903 genes had at least one of the 4685 SNP markers physically linked to the gene.

Thus, we have discovered a total of 3048 T2D genes, in which any genetic marker can be used to predict T2D, and thus these markers can be used as part of molecular diagnostic tests of T2D predisposition. In addition, we have disclosed a set of 4685 SNP markers which are predictive of T2D. The markers can also be used as part of pharmacogenetic tests which predict the efficacy and adverse reactions of antihyperglicemic agents and compounds. The genes discovered are also targets to new therapies of T2D, such as drugs. Other therapies are molecular, including gene transfer. The new genes can also be used to develop and produce new transgenic animals for studies of antihypertensive agents and compounds.

While this invention has been particularly shown and described with reference to preferred embodiments thereof, it will be understood by those skilled in the art that various changes in form and details may be made therein without departing from the scope of the invention as defined by the appended claims.

### References

Altshuler D, Hirschhorn JN, Klannemark M, Lindgren CM, Vohl MC, et al. 2000. The common PPARgamma Pro 12Ala polymorphism is associated with decreased risk of type 2 diabetes. Nat. Genet. 26:76-80.
American Diabetes Association. 2003. Report of the Expert Committee on the Diagnosis and Classification of Diabetes Mellitus. Diabetes Care 26:S5-S20.
Bamett AH, Eff C, Leslie RD, Pyke DA. 1981. Diabetes in identical twins. A study of 200 pairs. Diabetologia 20:87-93.
Bell GI, Xiang K, Newman MV, Wu S, Wright LG, Fajans SS, Spielman RS, Cox NJ. 1991. Gene for non-insulin-dependent diabetes mellitus (maturity-onset diabetes of the young subtype) is linked to DNA polymorphism on human chromosome 20q. Proc Natl Acad Sci 88:1484-1488.
Berg AH, Combs TP, Scherer PE. 2002. ACRP30/adiponectin: an adipokine regulating glucose and lipid metabolism. Trends Endocrinol Metab. 13:84-9.
Bierer B, Coligan JE, Margulies DH, Shevach EM, Strober W. 2002. Current Protocols in Immunology. NY:John Wiley & Sons.
Boden G. 2001. Free fatty acids-the link between obesity and insulin resistance. Endocr Pract. 7:44-51.
Buchanan TA.2003. Pancreatic beta-cell loss and preservation in type 2 diabetes. Clin Ther. 25 Suppl B:B32-46.
Byrne MM, Sturis J, Menzel S, Yamagata K, Fajans SS, Dronsfield MJ, Bain SC, Hattersley AT, Velho G, Froguel P, Bell GI, Polonsky KS. 1996. Altered insulin secretory response to glucose in diabetic and nondiabetic subjects with mutations in the diabetes susceptibility gene MODY3 on chromosome 12. Diabetes 45:1503-1510.
Chiu KC, Province MA, Dowse GK, Zimmet PZ, Wagner G, et al. 1992. A genetic marker at the glucokinase gene locus for type 2 (non-insulin-dependent) diabetes mellitus in Mauritian Creoles. Diabetologia 35:632-38.
Clement K, Pueyo ME, Vaxillaire M, Rakotoambinina B, Thuillier F, Passa P, Froguel P, Roberts J, Velho G. 1996. Assessment of insulin sensitivity in glucokinase-deficient subjects. Diabetologia 39:82-90.
Cole SP, Campling BG, Atlaw T, Kozbor D, Roder JC. 1984. Human monoclonal antibodies. Mol Cell Biochem. 62:109-20.
Damcott CM, Moffett SP, Feingold E, Barmada MM, Marshall JA, Hamman RF, Ferrell RE. 2004. Genetic variation in fatty acid-binding protein-4 and peroxisome proliferator-activated receptor gamma interactively influence insulin sensitivity and body composition in males. Metabolism. 53:303-9.
Dandona P, Aljada A, Bandyopadhyay A. 2004. Inflammation: the link between insulin resistance, obesity and diabetes. Trends Immunol. 25:4-7.
Deeb SS, Fajas L, Nemoto M, Pihlajamaki J, Mykkanen L, et al. 1998. A Pro12Ala substitution in PPARgamma2 associated with decreased receptor activity, lower body mass index and improved insulin sensitivity. Nat. Genet. 20:284-87.
Demenais F, Kanninen T, Lindgren CM, Wiltshire S, Gaget S, Dandrieux C, Almgren P, Sjogren M, Hattersley A, Dina C, Tuomi T, McCarthy MI, Froguel P, Groop LC. 2003. A meta-analysis of four European genome screens (GIFT Consortium) shows evidence for a novel region on chromosome 17p11.2-q22 linked to type 2 diabetes. Hum Mol Genet. 12:1865-73.
Dickson LM, Rhodes CJ. 2004. Pancreatic beta-cell growth and survival in the onset of type 2 diabetes: a role for protein kinase B in the Akt? Am J Physiol Endocrinol Metab. 287:E192-8.
Eriksson JG, Lindi V, Uusitupa M, Forsen TJ, Laakso M, Osmond C, Barker DJ. 2002. The effects of the Pro12Ala polymorphism of the peroxisome proliferator-activated receptor-gamma2 gene on insulin sensitivity and insulin metabolism interact with size at birth. Diabetes. 51:2321-4.
Eronen L, Geerts F, Toivonen H. 2004. A Markov chain approach to reconstruction of long haplotypes. Pac Symp Biocomput.:104-15.
Florez JC, Hirschhorn J, Altshuler D. 2003. The inherited basis of diabetes mellitus: implications for the genetic analysis of complex traits. Annu Rev Genomics Hum Genet 4:257-91.
Froguel P, Vaxillaire M, Sun F, Velho G, Zouali H, Butel MO, Lesage S, Vionnet N, Clement K, Fougerousse F, et al. 1992. Close linkage of glucokinase locus on chromosome 7p to early-onset non-insulin-dependent diabetes mellitus. Nature 356:162-164.
Gloyn AL, Hashim Y, Ashcroft SJ, Ash-field R, Wiltshire S, Tumer RC. 2001. Association studies of variants in promoter and coding regions of beta-cell ATP-sensitive K-channel genes SUR1 and Kir6.2 with Type 2 diabetes mellitus (UKPDS 53). Diabetes Med. 18:206-12.
Gloyn AL, Weedon MN, Owen KR, Turner MJ, Knight BA, et al. 2003. Large-scale association studies of variants in genes encoding the pancreatic {beta}-cell KATP channel subunits Kir6.2 (KCNJ11) and SUR1 (ABCC8) confirm that the KCNJ11 E23K variant is associated with type 2 diabetes. Diabetes 52:568-72.
Gough SC, Saker PJ, Pritchard LE, Merriman TR, Merriman ME, et al. 1995. Mutation of the glucagon receptor gene and diabetes mellitus in the UK: association or founder effect? Hum. Mol. Genet. 4:1609-12.
Griffiths AD, Malmqvist M, Marks JD, Bye JM, Embleton MJ, McCafferty J, Baier M, Holliger KP, Gorick BD, Hughes-Jones NC, et al. 1993. Human anti-self antibodies with high specificity from phage display libraries. EMBO J. 12:725-34.
   Gunton JE, Kulkarni RN, Yim S, Okada T, Hawthorne WJ, Tseng YH, Roberson RS, Ricordi C, O'Connell PJ, Gonzalez FJ, Kahn CR. 2005. Loss of ARNT/HIF 1 beta mediates altered gene expression and pancreatic-islet dysfunction in human type 2 diabetes. Cell. 122: 337-49.
Guthrie RA, Guthrie DW. 2004. Pathophysiology of diabetes mellitus. Crit Care Nurs Q. 27:113-25.
Hager J, Hansen L, Vaisse C, Vionnet N, Philippi A, et al. 1995. A missense mutation in the glucagon receptor gene is associated with non-insulin-dependent diabetes mellitus. Nat. Genet. 9:299-304.
Hani EH, Boutin P, Durand E, Inoue H, Permutt MA, et al. 1998. Missense mutations in the pancreatic islet beta cell inwardly rectifying K+ channel gene (KIR6.2/BIR): a meta-analysis suggests a role in the polygenic basis of Type II diabetes mellitus in Caucasians. Diabetologia 41:1511-15.
Hani EH, Clement K, Velho G, Vionnet N, Hager J, et al. 1997. Genetic studies of the sulfonylurea receptor gene locus in NIDDM and in morbid obesity among French Caucasians. Diabetes 46:688-94.
Hansen T, Echwald SM, Hansen L, Moller AM, Almind K, et al. 1998. Decreased tolbutamide-stimulated insulin secretion in healthy subjects with sequence variants in the high-affinity sulfonylurea receptor gene. Diabetes 47: 598-605.
Hara K, Okada T, Tobe K, Yasuda K, Mori Y, et al. 2000. The Pro12Ala polymorphism in PPAR gamma2 may confer resistance to type 2 diabetes. Biochem. Biophys. Res. Commun. 271:212-16.
Hay BN, Sorge JA, Shopes B. 1992. Bacteriophage cloning and Escherichia coli expression of a human IgM Fab. Hum Antibodies Hybridomas. 3:81-5.
Hayashi N, Kipriyanov S, Fuchs P, Welschof M, Dorsam H, Little M. 1995. A single expression system for the display, purification and conjugation of single-chain antibodies. Gene. 160:129-30.
Hering S, Karawajew L, Pasternak G. 1988. Raji-K562 hybrids and their use for trioma production. Biomed Biochim Acta. 47:211-6.
Herman WH, Fajans SS, Oritz FJ, Smith MJ, Sturis J, Bell GI, Polonsky KS, Halter JB. 1994. Abnormal insulin secretion, not insulin resistance, is the genetic or primary defect of MODY in the RW pedigree. Diabetes 43:40-46.
Huse WD, Sastry L, Iverson SA, Kang AS, Alting-Mees M, Burton DR, Benkovic SJ, Lemer RA. 1989. Generation of a large combinatorial library of the immunoglobulin repertoire in phage lambda. Science. 246:1275-81.
Inoue H, Ferrer J, Welling CM, Elbein SC, Hoffman M, et al. 1996. Sequence variants in the sulfonylurea receptor (SUR) gene are associated with NIDDM in Caucasians. Diabetes 45:825-31.
Kadowaki T, Kadowaki H, Mori Y, Tobe K, Sakuta R, Suzuki Y, Tanabe Y, Sakura H, Awata T, Goto Y, et al. 1994. A subtype of diabetes mellitus associated with a mutation of mitochondrial DNA. N Engl J Med 330:962-968.
Kahn CR, Flier JS, Bar RS, Archer JA, Gorden P, Martin MM, Roth J. 1976. The syndromes of insulin resistance and acanthosis nigricans. N Engl J Med 294:739-745.
Kohler G, Milstein C. 1975. Continuous cultures of fused cells secreting antibody of predefined specificity. Nature 256:495-497.
Kozbor D, Lagarde AE, Roder JC. 1982. Human hybridomas constructed with antigen-specific Epstein-Barr virus-transformed cell lines. Proc Natl Acad Sci U S A. 79:6651-5. Kwok P-Y. 2001. Methods for genotyping single nucleotide polymorphisms. Ann Rev Genomics Hum Genet. 2:235-258.
Laaksonen DE, Lakka TA, Lakka H-M, Nyyssönen K, Rissanen T, Niskanen LK, Salonen JT. 2002. Serum fatty acid composition predicts development of impaired fasting glycaemia and diabetes in middle-aged men. Diabetic Medicine 19:456-64.
Lakka H-M, Oksanen L, Tuomainen T-P, Kontula K, Salonen JT. 2000. The common pentanucleotide polymorphism of the 3'-untranslated region of the leptin receptor gene is associated with serum insulin levels and the risk of type 2 diabetes in non-diabetic men: a prospective case-control study. J Int Med 248: 77-83.
Lehtovirta M, Kaprio J, Grrop L, trombetta M, Bonadonna RC. 2005. Heritability of model-derived parameters of beta cell secretion during intravenous and oral glucose tolerance tests: a study of twins. Diabetologia 48: 1604-13.
Li SR, Baroni MG, Oelbaum RS, Stock J, Galton DJ. 1988. Association of genetic variant of the glucose transporter with non-insulin-dependent diabetes mellitus. Lancet 2:368-70.
Lowe WL Jr. Genetics of diabetes mellitus. Kluwer Academic Publishers, 2001.
Luan J, Browne PO, Harding AH, Halsall DJ, O'Rahilly S, Chatterjee VK, Wareham NJ. 2001. Evidence for gene-nutrient interaction at the PPARgamma locus. Diabetes. 50:686-9.
Martin BC, Warram JH, Krolewski AS, Bergman RN, Soeldner JS, Kahn CR. 1992. Role of glucose and insulin resistance in development of type 2 diabetes mellitus: results of a 25-year follow-up study. Lancet 340:925-9.
McCarthy MI, Hitman GA, Hitchins M, Riikonen A, Stengard J, et al. 1994. Glucokinase gene polymorphisms: a genetic marker for glucose intolerance in a cohort of elderly Finnish men. Diabetes Med. 11:198-204.
Memisoglu A, Hu FB, Hankinson SE, Manson JE, De Vivo I, Willett WC, Hunter DJ. 2003. Interaction between a peroxisome proliferator-activated receptor gamma gene polymorphism and dietary fat intake in relation to body mass. Hum Mol Genet. 12:2923-9.
Mori H, Ikegami H, Kawaguchi Y, Seino S, Yokoi N, et al. 2001. The Pro12 → Ala substitution in PPAR-gamma is associated with resistance to development of diabetes in the general population: possible involvement in impairment of insulin secretion in individuals with type 2 diabetes. Diabetes 50:891-94.
Motoshima H, Wu X, Sinha MK, Hardy VE, Rosato EL, Barbot DJ, Rosato FE, Goldstein BJ. 2002. Differential regulation ofadiponectin secretion from cultured human omental and subcutaneous adipocytes: effects of insulin and rosiglitazone. J Clin Endocrinol Metab. 87:5662-7.
Nielsen PE, Egholm M, Berg RH, Buchardt O. 1991. Sequence-selective recognition of DNA by strand displacement with a thymine-substituted polyamide. Science. 254:1497-500.
Peltonen L, Jalanko A, Varilo T. Molecular genetics of the Finnish disease heritage. 1999. Hum Mol Genet. 8: 1913-23.
Pontiroli AE, Capra F, Veglia F, Ferrari M, Xiang KS, et al. 1996. Genetic contribution of polymorphism of the GLUT1 and GLUT4 genes to the susceptibility to type 2 (non-insulin-dependent) diabetes mellitus in different populations. Acta Diabetologica 33:193-97.
Poulsen P, Kyvik KO, Vaag A, Beck-Nielsen H. 1999. Heritability of type II (non-insulin-dependent) diabetes mellitus and abnormal glucose tolerance-a population-based twin study. Diabetologia 42:139-45.
Prentki M, Joly E, El-Assaad W, Roduit R. 2002. Malonyl-CoA signaling, lipid partitioning, and glucolipotoxicity: role in beta-cell adaptation and failure in the etiology of diabetes. Diabetes. 51 Suppl 3:S405-13.
Ravussin E, Smith SR. 2002. Increased fat intake, impaired fat oxidation, and failure of fat cell proliferation result in ectopic fat storage, insulin resistance, and type 2 diabetes mellitus. Ann N Y Acad Sci. 967:363-78.
Reardon W, Ross RJM, Sweeney MG, Luxon LM, Pembrey ME, Harding AE, Trembath RC. 1992. Diabetes mellitus associated with a pathogenic point mutation in mitochondrial DNA. Lancet 340:1376-1379.
Sajantila A, Salem AH, Savolainen P, Bauer K, Gierig C, Paabo S. 1996. Paternal and maternal DNA lineages reveal a bottleneck in the founding of the Finnish population. Proc Natl Acad Sci U S A. 93: 12035-9.
Salonen JT, Lakka TA, Lakka H-M, Valkonen V-P, Everson SA, Kaplan GA. 1998. Hyperinsulinemia is associated with the Incidence of hypertension and dyslipidemia in middle-aged men. Diabetes 47:270-275.
Salonen JT, Malin R, Tuomainen TP, Nyyssonen K, Lakka TA, Lehtimaki T. 1999. Polymorphism in high density lipoprotein paraoxonase gene and risk of acute myocardial infarction in men: prospective nested case-control study. BMJ. 319:487-9.
Salonen JT, Tuomainen T-P, Kontula K. 2000. Role of C282Y mutation in haemochromatosis gene in development of type 2 diabetes in healthy men: prospective cohort study. Brit Med J 320: 1706-1707.
Salonen JT. 1988. Is there a continuing need for longitudinal epidemiologic research? The Kuopio Ischaemic Heart Disease Risk Factor Study. Ann Clin Res 20: 46-50.
Salonen, JT. 2003. The value of founder populations in genetic analysis. Current Drug Discovery pp.27, 30-31.
Saltiel AR, Kahn CR. 2001. Insulin signalling and the regulation of glucose and lipid metabolism. Nature. 414:799-806.
Scheen AJ. 2003. Pathophysiology of type 2 diabetes. Acta Clin Belg. 58:335-41.
Scheen AJ. 2004. Pathophysiology of insulin secretion. Ann Endocrinol (Paris). 65:29-36.
Shulman GI. 2000. Cellular mechanisms of insulin resistance. J Clin Invest. 106:171-6.
Syvänen A-C. 2001. Accessing genetic variation: Genotyping single nucleotide polymorphisms. Nature Reviews Genetics. 2:930-942.
Takekawa K, Ikegami H, Fukuda M, Ueda H, Kawaguchi Y, et al. 1994. Early-onset type 2 (non-insulin-dependent) diabetes mellitus is associated with glucokinase locus, but not with adenosine deaminase locus, in the Japanese population. Diabetes Res. Clin. Pract. 23:141-46.
Tao T, Tanizawa Y, Matsutani A, Matsubara A, Kaneko T, Kaku K. 1995. HepG2/erythrocyte glucose transporter (GLUT1) gene in NIDDM: a population association study and molecular scanning in Japanese subjects. Diabetologia 38:942-47.
Taylor SI. 1992. Lilly Lecture: molecular mechanisms of insulin resistance: lessons from patients with mutations in the insulin-receptor gene. Diabetes 41:1473-1490.
Toivonen HT, Onkamo P, Vasko K, Ollikainen V, Sevon P, Mannila H, Herr M, Kere J. 2000. Data mining applied to linkage disequilibrium mapping. Am J Hum Genet. 67:133-45.
Tomas E, Tsao TS, Saha AK, Murrey HE, Zhang Cc C, Itani SI, Lodish HF, Ruderman NB. 2002. Enhanced muscle fat oxidation and glucose transport by ACRP30 globular domain: acetyl-CoA carboxylase inhibition and AMP-activated protein kinase activation. Proc Natl Acad Sci U S A. 99:16309-13.
Tuomainen TP, Kontula K, Nyyssonen K, Lakka TA, Helio T, Salonen JT. 1999. Increased risk of acute myocardial infarction in carriers of the hemochromatosis gene Cys282Tyr mutation : a prospective cohort study in men in eastern Finland. Circulation. 100:1274-9.
Uimari P, Kontkanen O, Visscher PM, Pirskanen M, Fuentes R, Salonen JT. 2005. Genome-wide linkage disequilibrium from 100,000 SNPs in the East Finland founder population. Twin Res Hum Genet. 8:185-97.
van den Ouwenland JMW, Lemkes HHPJ, Ruitenbeek W, Sandkuijl LA, de Vijlder MF, Struyvenberg PAA, van de Kamp, Maassen JA. 1992. Mutation in mitochondrial tRNA (Leu(URR) gene in a large pedigree with maternally transmitted type II diabetes mellitus and deafness. Nature Genet 1:368-371.
Vaxillaire M, Boccio V, Philippi A, Vigouroux C, Terwilliger J, Passa P, Beckman JS, Velho G, Lathrop GM, Froguel P. 1995. A gene for maturity onset diabetes of the young (MODY) maps to chromosome 12q. Nature Genet 9:418-423.
Vionnet N, Stoffel M, Takeda J, Yasuda K, Bell GI, Zouali H, Lesage S, Velho G, Iris F, Passa P, et al. 1992. Nonsense mutation in the glucokinase gene causes early-onset non-insulin-dependent diabetes mellitus. Nature 356:721-722.
World Health Organization and the International Diabetes Federation. 2004. Diabetes Action Now booklet. http://www.who.int/diabetes/actionnow/booklet/en (Accessed 02.07.04)
World Health Organization. 2004. Diabetes Mellitus fact sheet. http://www.who.int/mediacentre/factsheets/fs138/en (Accessed 02.07.04).
Wu X, Hoffstedt J, Deeb W, Singh R, Sedkova N, Zilbering A, Zhu L, Park PK, Arner P, Goldstein BJ. 2001. Depot-specific variation in protein-tyrosine phosphatase activities in human omental and subcutaneous adipose tissue: a potential contribution to differential insulin sensitivity. J Clin Endocrinol Metab. 86:5973-80.
Yamagata K, Furuta H, Oda N, Kaisaki PJ, Menzel S, Cox NJ, Fajans SS, Signorini S, Stoffel M, Bell GI. 1996. Mutations in the hepatocyte factor-4α gene in maturity-onset diabetes of the young (MODY 1). Nature 384:458-460.
Yamagata K, Oda N, Kaisaki PJ, Menzel S, Furuta H, Vaxillaire M, Southarm L, Cox RD, Lathrop GM, Boriraj W, Chen X, Cox NJ, Oda Y, Yano H, Le Beau MM, Yamada S, Nishigori H, Takeda J, Fajans SS, Hattersley AT, Iwasaki N, Hansen T, Pedersen O, Polonsky KS, Bell GI. 1996. Mutations in the hepatocyte nuclear factor-1α gene in maturity-onset diabetes of the young (MODY 3). Nature 384:455-458.
Yamauchi T, Kamon J, Minokoshi Y, Ito Y, Waki H, Uchida S, Yamashita S, Noda M, Kita S, Ueki K, Eto K, Akanuma Y, Froguel P, Foufelle F, Ferre P, Carling D, Kimura S, Nagai R, Kahn BB, Kadowaki T. 2002. Adiponectin stimulates glucose utilization and fatty-acid oxidation by activating AMP-activated protein kinase. Nat Med. 8:1288-95.
Zick Y. 2001. Insulin resistance: a phosphorylation-based uncoupling of insulin signaling. Trends Cell Biol. 11:437-41.

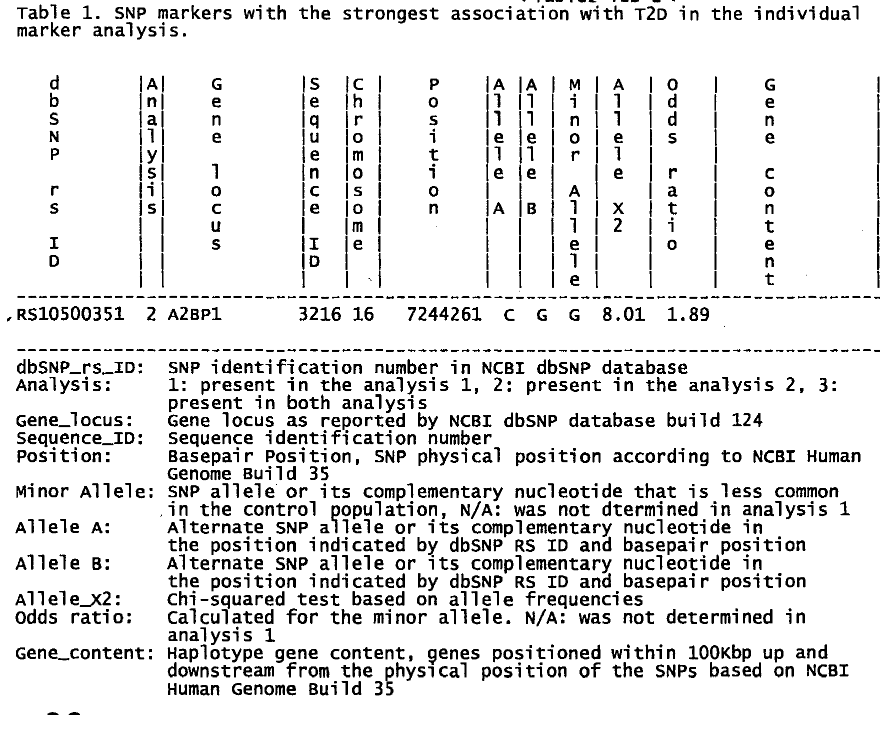

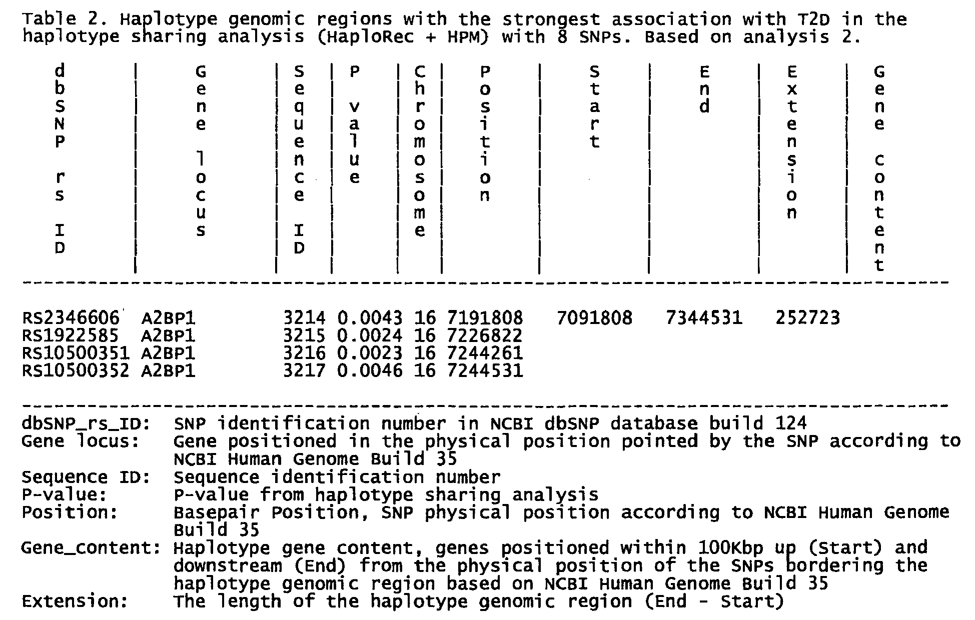

## Claims

1. A method for diagnosing a predisposition for type 2 diabetes mellitus (T2D) in a human subject comprising:
a) providing a biological sample taken from the subject,
b) genotyping SNP markers
rs10500351 in said sample, wherein said SNP markers is associated with gene A2BP1,and
c) comparing SNP marker data obtained in step b) to SNP marker data from healthy and diseased people to diagnose a predisposition for T2D.

2. The method according to claim 1 further comprising a marker set to assess the ancestry of a subject.

3. The method according to claim 2, wherein a microsatellite marker set or SNP marker set is used to assess the ancestry of an individual.

4. The method according to claim 1 further comprising a step of combining non-genetic information with the SNP marker data to diagnose a predisposition for T2D.

5. The method according to claim 4, wherein the non-genetic information concerns age, gender, ethnicity, socioeconomic status, history of gestational diabetes, other medical history of the subject, family history of relevant conditions, psychological traits and states, behaviour patterns and habits, biochemical measurements, clinical measurements, and measurements of obesity and adiposity.

6. The method according to claim 5, wherein the other medical history of the subject concerns the metabolic syndrome, glucose intolerance, increased insulin resistance, obesity, nephropathies, hypothyroidism, hyperthyroidism, disorders of the pituitary gland, disorders of the hypothalamus, disorders of the pancreas, appetite and eating disorders and conditions which limit physical activity, low weight at birth and/or premature birth.

7. The method according to claim 5 or 6, wherein the relevant family history information concerns type 1 and type 2 diabetes, gestational diabetes, other type of diabetes, the metabolic syndrome, glucose intolerance, increased insulin resistance, obesity, hypothyroidism, hyperthyroidism, disorders of the pituitary grand, disorders of the hypothalamus, disorders of the pancreas and appetite and eating disorders.

8. The method according to any one of the claims 5 to 7, wherein the biochemical measurements include measurements of lipids, lipoproteins, carbohydrates and peptides and proteins in human tissues and body fluids.

9. The method according to claim 8, wherein the protein measurements include the measurements of prohormones, hormones, enzymes and receptors.

10. The method according to claim 8 or 9, wherein the measurements include the measurements of glycated peptides and proteins, advanced glycated end products, oxidatively modified proteins and peptides, glucagons, glucagons-like peptides (GLP), other insulinotropic peptides, proinsulins, insulin, insulin degrading enzymes, growth hormone, thyrotropin-releasing hormone (TRH), TRH-like peptides, prolactine, amylins, homocysteine, C-peptide, leptins, adiponectins, ghrelins, gastrins, resistin, obestatin, incretins, markers of mild chronic inflammation, such as TNFα, IL-6 and C-reactive protein, dipeptidyl peptidase IV, endothelins, pituitary adenylate cyclase activating peptides (PACAPs), vasoactive intestinal peptides (VIPs), hypothalamic regulatory peptides, opioid peptides, neuropeptide Y, adrenomedullin, atrial and brain natriuretic peptides (ANPs, BNPs), heat shock protein derived peptides, ferritin, transferrin, ceruloplasmin, albumin, the endogenous activators, inhibitors, inactivators, receptors and degradators of the said peptides and enzymes involved in the synthesis and release of the said peptides.

11. The method according to claim 5, wherein the measurements of obesity and adiposity include height, weight, body-mass index (kg/m2), waist circumference, waist-to-hip circumference ratio, skinfold thickness measurements, adipose tissue thickness measurements and measurements of amount and proportion of adipose tissue of the body.

12. The method according to claim 5, wherein the behaviour patterns and habits include tobacco smoking, physical activity, dietary intakes of nutrients, salt intake, alcohol intake and consumption patterns and coffee consumption and quality.

## Patentansprüche

1. Verfahren zum Diagnostizieren einer Prädisposition für Typ-2-Diabetes mellitus (T2D) bei einem humanen Patienten, umfassend:
a) Bereitstellung einer vom Patienten entnommenen biologischen Probe,
b) Genotypisierung des SNP-Markers rs10500351 in der Probe, wobei der SNP-Marker mit Gen A2BP1 assoziiert ist, und
c) Vergleich von in Schritt b) erhaltenen SNP-Markerdaten mit SNP-Markerdaten von gesunden und kranken Personen zum Diagnostizieren einer Prädisposition für T2D.

2. Verfahren nach Anspruch 1, ferner umfassend ein Markerset zur Beurteilung der Abstammung eines Patienten.

3. Verfahren nach Anspruch 2, wobei ein Mikrosatelliten-Markerset oder SNP-Markerset zur Beurteilung der Abstammung eines Individuums verwendet wird.

4. Verfahren nach Anspruch 1, ferner umfassend einen Schritt zum Kombinieren von nicht genetischer Information mit den SNP-Markerdaten zum Diagnostizieren einer Prädisposition für T2D.

5. Verfahren nach Anspruch 4, wobei die nicht genetische Information das Alter, das Geschlecht, die Ethnizität, den sozioökonomischen Status, die Anamnese eines Gestationsdiabetes, die sonstige medizinische Vorgeschichte des Patienten, die Familienanamnese relevanter Erkrankungen, die psychologischen Merkmale und Zustände, die Verhaltensmuster und Gewohnheiten, die biochemischen Messungen, die klinischen Messungen und Messungen von Obesitas und Adipositas betrifft.

6. Verfahren nach Anspruch 5, wobei die sonstige medizinische Vorgeschichte des Patienten das Metabolische Syndrom, Glucoseintoleranz, erhöhte Insulinresistenz, Obesitas, Nephropathien, Hypothyreoidismus, Hyperthyreoidismus, hypophysäre Störungen, hypothalamische Störungen, pankreatische Störungen, Appetit- und Essstörungen und Zustände, welche die körperliche Aktivität einschränken, ein niedriges Geburtsgewicht und/oder eine Frühgeburt betrifft.

7. Verfahren nach Anspruch 5 oder 6, wobei die relevante Information zur Familienanamnese Typ-1- und Typ-2-Diabetes, Gestationsdiabetes, einen anderen Diabetestyp, das Metabolische Syndrom, Glucoseintoleranz, erhöhte Insulinresistenz, Obesitas, Hypothyreoidismus, Hyperthyreoidismus, hypophysäre Störungen, hypothalamische Störungen, pankreatische Störungen und Appetit- und Essstörungen betrifft.

8. Verfahren nach einem der Ansprüche 5 bis 7, wobei die biochemischen Messungen Messungen von Lipiden, Lipoproteinen, Kohlenhydraten und Peptiden und Proteinen in humanen Geweben und Körperflüssigkeiten einschließen.

9. Verfahren nach Anspruch 8, wobei die Proteinmessungen die Messungen von Prohormonen, Hormonen, Enzymen und Rezeptoren einschließen.

10. Verfahren nach Anspruch 8 oder 9, wobei die Messungen die Messungen von glykierten Peptiden und Proteinen, fortgeschrittenen glykierten Endprodukten, oxidativ modifizierten Proteinen und Peptiden, Glucagonen, Glucagon-ähnlichen Peptiden (GLP), anderen insulinotropen Peptiden, Proinsulinen, Insulin, Insulinabbauenden Enzymen, Wachstumshormon, Thyreotropinfreisetzendem Hormon (TRH), TRH-ähnlichen Peptiden, Prolactin, Amylinen, Homocystein, C-Peptid, Leptinen, Adiponectinen, Ghrelinen, Gastrinen, Resistin, Obestatin, Inkretinen, Markern einer leichten chronischen Entzündung, wie zum Beispiel TNFa, IL-6 und C-reaktivem Protein, Dipeptidylpeptidase IV, Endothelinen, Hypophysen-Adenylatcyclase-aktivierenden Peptiden (PACAP), vasoaktiven Intestinalpeptiden (VIP),
regulatorischen Hypothalamus-Peptiden, Opioidpeptiden, Neuropeptid Y, Adrenomedullin, atrialen und Hirnnatriuretischen Peptiden (ANP, BNP), Hitzeschockproteinabgeleiteten Peptiden, Ferritin, Transferrin, Ceruloplasmin, Albumin, den endogenen Aktivatoren, Inhibitoren, Inaktivatoren, Rezeptoren und Degradatoren der Peptide und an der Synthese beteiligten Enzymen und Freisetzung der Peptide einschließen.

11. Verfahren nach Anspruch 5, wobei die Messungen von Obesitas und Adipositas die Größe, das Gewicht, den Körpermasseindex (kg/m²), den Taillenumfang, das Taillen-/Hüftumfang-Verhältnis, Hautfaltendickenmessungen, Messungen der Fettgewebedicke und Messungen der Menge und des Anteils an Körperfettgewebe einschließen.

12. Verfahren nach Anspruch 5, wobei die Verhaltensmuster und Gewohnheiten Tabakrauchen, körperliche Aktivität, diätetische Aufnahmen von Nährstoffen, Salzkonsum, Alkoholkonsum und -verbrauchsmuster und Kaffeegenuss und -qualität einschließen.

## Revendications

1. Méthode de diagnostic d'une prédisposition au diabète sucré de type 2 (T2D) chez un sujet humain comprenant :
a) l'obtention d'un échantillon biologique prélevé chez le sujet,
b) le génotypage du marqueur SNP rs10500351 dans ledit échantillon, ledit marqueur SNP étant associé au gène A2BP1, et
c) la comparaison des données du marqueur SNP obtenues à l'étape b) avec les données du marqueur SNP de personnes saines et malades pour diagnostiquer une prédisposition au T2D.

2. Méthode selon la revendication 1, comprenant en outre un ensemble de marqueurs pour évaluer l'ascendance d'un sujet.

3. Méthode selon la revendication 2, **caractérisée en ce qu'**on utilise un ensemble de marqueurs microsatellites ou un ensemble de marqueurs SNP pour évaluer l'ascendance d'un individu.

4. Méthode selon la revendication 1, comprenant en outre une étape consistant à combiner des informations non génétiques avec les données du marqueur SNP pour diagnostiquer une prédisposition au T2D.

5. Méthode selon la revendication 4, **caractérisée en ce que** les informations non génétiques concernent l'âge, le sexe, l'ethnicité, le statut socio-économique, l'antécédent de diabète gestationnel, d'autres antécédents médicaux du sujet, l'antécédent familial d'affections pertinentes, les traits et les états psychologiques, les comportements et les habitudes, les mesures biochimiques, les mesures cliniques et les mesures de l'obésité et de l'adiposité.

6. Méthode selon la revendication 5, **caractérisée en ce que** les autres antécédents médicaux du sujet concernent le syndrome métabolique, l'intolérance au glucose, la résistance accrue à l'insuline, l'obésité, les néphropathies, l'hypothyroïdisme, l'hyperthyroïdisme, les troubles de l'hypaphyse, les troubles de l'hypothalamus, les troubles du pancréas, les troubles de l'appétit et du comportement alimentaire et les affections qui limitent l'activité physique, un poids faible à la naissance et/ou l'accouchement prématuré.

7. Méthode selon la revendication 5 ou 6, **caractérisée en ce que** les antécédents familiaux pertinents concernent le diabète de type 1 et de type 2, le diabète gestationnel, d'autres types de diabètes, le syndrome métabolique, l'intolérance au glucose, la résistance accrue à l'insuline, l'obésité, l'hypothyroïdisme, l'hyperthyroïdisme, les troubles de l'hypophyse, les troubles de l'hypothalamus, les troubles du pancréas et les troubles de l'appétit et du comportement alimentaire.

8. Méthode selon l'une quelconque des revendications 5 à 7, **caractérisée en ce que** les mesures biochimiques incluent les mesures des lipides, des lipoprotéines, des glucides et des peptides et protéines dans les tissus humains et les liquides corporels.

9. Méthode selon la revendication 8, **caractérisée en ce que** les mesures de protéines incluent les mesures des prohormones, des hormones, des enzymes et des récepteurs.

10. Méthode selon la revendication 8 ou 9, **caractérisée en ce que** les mesures incluent les mesures des peptides et protéines glyqués, des produits finaux glyqués avancés, des protéines et peptides modifiés par voie oxydative, des glucagons, des peptides de type glucagons (GLP), d'autres peptides insulinotropes, des proinsulines, de l'insuline, des enzymes de dégradation de l'insuline, de l'hormone de croissance, de l'hormone de libération de la thyrotropine (TRH), des peptides de type TRH, de la prolactine, des amylines, de l'homoeystéine, du peptide C, des leptines, des adiponectines, des ghrélines, des gastrines, de la résistine, de l'obestatine, des incrétines, des marqueurs d'inflammations chroniques légères, tels que le TNFα, l'IL-6 et la protéine C réactive, de la dipeptidyl-peptidase IV, des endothélines, des peptides d'activation de l'adénylate cyclase pituitaire (PACAP), des peptides intestinaux vasoactifs (VIP), des peptides régulateurs hypothalamiques, des peptides opioïdes, du neuropeptide Y, de l'adrénomédulline, des peptides natriurétiques auriculaires et cérébraux (ANP, BNP), des peptides issus de protéines de chocs thermiques, de la ferritine, de la transferrine, de la céruloplasmine, de l'albumine, des activateurs, inhibiteurs, désactivateurs, récepteurs et agents de dégradation endogènes desdits peptides et enzymes impliqués dans la synthèse et la libération desdits peptides.

11. Méthode selon la revendication 5, **caractérisée en ce que** les mesures d'obésité et d'adiposité incluent des mesures de la taille, du poids, de l'index de masse corporelle (kg/m2), du tour de taille, du rapport tour de taille-tour de hanche, de l'épaisseur du pli cutané, des mesures de l'épaisseur du tissu adipeux et des mesures de la quantité et de la proportion du tissu adipeux de l'organisme.

12. Méthode selon la revendication 5, **caractérisée en ce que** les comportements et les habitudes incluent le tabagisme, l'activité physique, les prises alimentaires de nutriments, la prise de sel, la prise d'alcools et les modes de consommation et la consommation et qualité du café.
